(19) Europäisches Patentamt

European Patent Office

Office européen des brevets

(11) **EP 1 008 845 A1**

(12) **EUROPÄISCHE PATENTANMELDUNG**

(43) Veröffentlichungstag:
**14.06.2000 Patentblatt 2000/24**

(51) Int. Cl.[7]: **G01N 21/64**, G02B 21/06,
C12Q 1/68

(21) Anmeldenummer: **98123687.0**

(22) Anmeldetag: **11.12.1998**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(71) Anmelder:
**Ruprecht-Karls-Universität Heidelberg
69117 Heidelberg (DE)**

(72) Erfinder:
• **Huasmann, Michael
67071 Ludwigshafen (DE)**

• **Cremer, Christoph
69126 Heidelberg (DE)**
• **Bradl, Joachim
69198 Schriesheim (DE)**
• **Schneider, Bernhard
67346 Speyer (DE)**

(74) Vertreter: **Rudolph, Ulrike, Dr.
Patentanwältin
In der Schanz 10
69198 Schriesheim (DE)**

(54) **Wellenfeldmikroskop, Wellenfeldmikroskopieverfahren, auch zur DNA-Sequenzierung, und Kalibrierverfahren für die Wellenfeldmikroskopie**

(57) Die Erfindung betrifft zwei neue Wellenfeldmikroskope Typ I und Typ II, die sich dadurch auszeichnen, daß sie jeweils ein Anregungs- und Beleuchtungssystem umfassen, das wenigstens eine reelle und eine virtuelle Beleuchtungsquelle und wenigstens ein Objektiv (im Fall von Typ II) bzw. zwei Objektive (im Fall von Typ I) umfaßt, wobei Beleuchtungsquellen und Objektiv(e) derart zueinander angeordnet sind, daß sie zur Erzeugung von ein- zwei und dreidimensionalen stehenden Wellenfeldern im Objektraum geeignet sind. Das erfindungsgemäße Kalibrierverfahren ist an diese Wellenfeldmikroskope angepaßt und erlaubt geometrische Distanzmessungen zwischen fluorochrommarkierten Objektstrukturen, deren Distanz geringer als die Halbwertsbreite des Hauptmaximums der effektiven Punktbildfunktion sein kann. Die Erfindung betrifft außerdem ein Verfahren zur wellenfeldmikroskopischen DNA-Sequenzierung.

EP 1 008 845 A1

Printed by Xerox (UK) Business Services
2.16.7 (HRS)/3.6

## Beschreibung

Die Erfindung betrifft ein Wellenfeldmikroskop mit einem Beleuchtungs- bzw. Anregungssystem, das wenigstens eine reelle und eine virtuelle Beleuchtungsquelle und wenigstens ein Objektiv umfaßt, wobei Beleuchtungsquellen und Objektiv (e) derart zueinander angeordnet sind, daß sie zur Erzeugung eines eindimensionalen stehenden Wellenfeldes geeignet sind, mit einem Objektraum, der Halte und Manövriervorrichtung(en) für ein Objekt umfaßt, und mit einem Detektionssystem, das ein Objektiv, ein Okular und einen Detektor umfaßt, sie betrifft außerdem ein daran angepaßtes Kalibrierverfahren für geometrische Distanzmessungen zwischen fluorochrommarkierten Objektstrukturen, deren Distanz geringer als die Halbwertsbreite des Hauptmaximums der effektiven Punktbildfunktion sein kann, und sie betrifft ein darauf aufbauendes Verfahren zur wellenfeldmikroskopischen DNA-Sequenzierung.

## Stand der Technik

Durch den Einsatz hochspezifischer Marker, wie z.B. DNA-Proben oder Protein-Sonden, ist es möglich, in biologischen (Mikro-)Objekten, insbesondere in Zellen, Zellkernen, Zellorganellen oder auf Chromosomen, - im folgenden vereinfacht Objekt genannt —, nahezu beliebig kleine (Sub-)Strukturen zu markieren. Es können Strukturen in Dimensionen von einigen um ($10^{-6}$ m) bis zu wenigen zehn nm ($10^{-9}$ m) spezifisch dargestellt werden. Die Marker sind üblicherweise mit Fluorochromen oder auch kolloidalen Mikro(gold-)partikel gekoppelt, um ihre optische Detektion und Abbildung zu erleichtern bzw. überhaupt erst zu ermöglichen.

Um zwei Marker innerhalb desselben Objekts separat voreinander detektieren zu können, sind die betreffenden Marker häufig mit verschiedenfarbigen Fluorochromen gekoppelt. Das zur Verfügung stehende Farbemissionsspektrum der üblicherweise verwendeten Fluorochrome reicht von tiefblau über grün, rot bis in den infraroten Spektralbereich. Es können aber auch Fluorochrome verwendet werden, die sich weder in ihrem Anregungsspektrum noch in ihrem Fluoreszenzspektrum unterscheiden, sondern bei denen die Lebensdauer ihrer Fluoreszenzemission als Parameter zur Unterscheidung genutzt wird. Letztere haben den Vorteil, daß wellenlängenabhängige fokale Shifts nicht auftreten. Fluorochrome können auch ein unterschiedliches Emissionsspektrum haben und damit verschiedene spektrale Signatur besitzen, aber mit derselben Photonenenergie anregbar sein, z.B. durch Mehrphotonenprozesse. Auch in diesem Fall können wellenlängenabhängige fokale Shifts in der Anregung zwischen Fluorochromen verschiedener spektraler Signatur vermieden werden.

Die vorstehend genannten, an spezifische (Sub-)Strukturen in biologischen Mikroobjekten bindbaren bzw. gebundenen Fluorochrome werden im folgenden als Fluoreszenzmarker bezeichnet.

Stimmen Anregungsspektrum und/oder Emissionsspektrum und/oder die Fluoreszenzlebensdauern zweier Fluoreszenzmarker überein, so haben diese Fluoreszenzmarker hinsichtlich des betreffenden Parameters die gleiche spektrale Signatur. Unterscheiden sich die Fluoreszenzmarker in einem oder mehreren für die Messung relevanten Parametern, so habe sie unterschiedliche spektrale Signatur.

Unter Fluoreszenz wird im folgenden jede Photonenwechselwirkung verstanden, bei der zwischen dem Anregungsspektrum und dem Emissionsspektrum desselben Stoffes Unterschiede auftreten, die nicht auf monochromatische Absorption oder Streuung zurückgeführt werden können. Dies schließt insbesondere auch Mehrphotonenwechselwirkungen ein, bei denen die Anregungswellenlängen größer sein können als die Emissionswellenlängen.

Ferner wird hier der Begriff Fluoreszenz auch für die eng damit verwandten Phänomene der Lumineszenz, insbesondere die Phosphoreszenz verwendet. Dies schließt insbesondere längere mittlere Fluoreszenzlebensdauern ein, z.B. Fluoreszenzlebensdauern im Bereich von bis zu mehreren oder vielen msec (Millisekunden). Im folgenden werden die eng verwandten Vorgänge der Lumineszenz, Phosphoreszenz und Fluoreszenz als gleichermaßen erfindungsrelevant angesehen.

Die Detektion und Abbildung von Fluoreszenzmarker in ausgedehnten biologischen Objekten und die quantitative Lokalisation bezüglich definierter Objektpunkte /Objektstrukturen (Distanz- und Winkelmessungen) wird mit lichtmikroskopischen Meßverfahren durchgeführt. Hierbei spielt die sog. "Punktbildfunktion" (point spread function =PSF) oder "Punktantwort" des verwendeten Mikroskops oder allgemein des optischen Systems, d.h. dessen Fähigkeit, von einem "ideal punktförmigen" Objekt ein ebenso ideal punktförmiges Abbild zu erzeugen, eine entscheidende Rolle. Die Punktbildfunktion ist ein charakteristisches Merkmal einer jeden abbildenden Optik und ein Maß für deren Qualität.

Distanzmessungen zwischen Objektstrukturen hängen wesentlich von der effektiven— d.h. der lokal im markierten Objektpunkt gegebenen - Punktbildfunktion ab. Diese effektive Punktbildfunktion wiederum hängt stark von der jeweiligen lokalen Brechzahl und der Absorption im Objekt, im Einbettungsmedium des Objektes, in der Immersionsflüssigkeit und gegebenenfalls in den Deckgläsern ab.

Die effektive Punktbildfunktion unterscheidet sich im allgemeinen deutlich von der für das verwendete Mikroskop berechneten Punktbildfunktion. Auch die unter technisch optimierten Randbedingungen gemessenen Punktbildfunktionen unterscheiden sich in der Regel von den unter praktischen Routinelaborbedingungen in biologischen Objekten erzielbaren effektiven Punktbildfunktionen.

Da diese effektiven Punktbildfunktionen meist nicht zur Verfügung stehen, greift man zur Kalibrierung der Distanzmessungen auf ideale, berechnete Ergebnisse zurück bzw. auf Kalibrierungsmessungen, die unter Standardbedingungen durchgeführt wurden, wie z.B. Reflexionsverfahren. Beide Verfahren gehen jedoch zu Lasten der Präzision bei der dreidimensionalen Distanzmessung in biologischen Mikroobjekten, und die Folge ist eine erhebliche Unsicherheit in der Bestimmung der tatsächlichen räumlichen Distanz zwischen den Objektstrukturen; bei biologischen Objekten beinhalten solche quantitativen Größenabschätzungen Unsicherheiten von bis zu mehreren Mikrometern.

Bis in die jüngste Zeit herrschte in der Fachwelt die praktisch einhellige Überzeugung, daß zwei Objektstrukturen nur dann separiert werden können, wenn sie mindestens eine Halbwertsbreite des Hauptmaximums der effektiven Punktbildfunktion von einander entfernt sind.

Erst 1996 gelang es den Urhebern der vorliegenden Erfindung, ein Kalibrierverfahren für die Fernfeldmikroskopie (und auch die Flußfluorometrie) bereitzustellen, mit dem es möglich ist, Distanzmessungen zwischen Objektstrukturen, deren Abstand voneinander geringer ist als das Auflösungsvermögen des betreffenden Fernfeldmikroskops, d.h. die weniger als die Halbwertsbreite des Hauptmaximums der effektiven Punktbildfunktion voneinander entfernt liegen, unabhängig von der Lage der betreffenden Objektstrukturen im dreidimensionalen Raum, mit hoher Genauigkeit durchzuführen.

Dieses Verfahren umfaßt die folgenden Schritte:

- Vor, während oder nach der Präparation des betreffenden Objekts auf bzw. in einem Objekthalter, insbesondere Objektträgerplättchen, Objektträgerfaser/-kapillare oder Objektträgerflüssigkeit, werden die zu untersuchenden bzw. zu ortenden Strukturen (Meßstrukturen) mit Fluoreszenzfarbstoffen verschiedener und/oder gleicher spektraler Signatur markiert, d.h. solche zu ortende Strukturen (Meßstrukturen), die sich in unmittelbarer Nähe zueinander, nämlich innerhalb der Halbwertsbreite des Hauptmaximums ihrer effektiven Punktbildfunktion, befinden, werden mit Fluoreszenzfarbstoffen verschiedener spektraler Signatur markiert, während solche Meßstrukturen, deren Abstand voneinander größer ist als die Halbwertsbreite des Hauptmaximums der effektiven Punktbildfunktion, mit Fluoreszenzfarbstoffen verschiedener oder gleicher spektraler Signatur markiert werden. Zwei zu ortende Meßstrukturen dürfen immer dann mit der gleichen spektralen Signatur markiert sein, wenn sie z.B. durch ihre relative Lage oder durch andere Kriterien eindeutig identifiziert werden können.
- Mit den gleichen Fluoreszenzfarbstoffen werden Kalibriertargets definierter Größe und räumlicher Anordnung markiert,

- die fluoreszierenden Kalibriertargets werden entweder zusammen mit den Objekten oder separat auf bzw. in dem bzw. einem Objekthalter (Objektträgerplättchen, Objektträgerfaser/-kapillare, Objektträgerflüssigkeit o.a.) präpariert.
- (Untersuchungs-)Objekt und Kalibriertargets werden unter übereinstimmenden Bedingungen, gleichzeitig oder nacheinander mikroskopisch oder flußfluorometrisch untersucht.
- Jeweils zwei definierte Kalibriertargets verschiedener spektraler Signatur werden unter Berücksichtigung des wellenlängenabhängigen Abbildungs- und Lokalisationsverhaltens des jeweiligen optischen Systems (Mikroskop oder Flußfluorometer) vermessen, die dabei ermittelten Meßwerte gleich Ist-Werte werden mit den vorbekannten tatsächlichen Distanzwerten gleich Soli-Werten (d.h. den aufgrund der Geometrie berechneten Soll-Lokalisationen) verglichen, und die Differenz zwischen Ist-Werten und Soll-Werten, nämlich der Kalibrierwert, wird zur Korrektur des durch das optische System bedingten Versatzes in der Detektion unterschiedlicher Emissionsloci insbesondere der Meßstrukturen verwendet.

Mit anderen Worten: Die Distanzmessung zwischen den (je nach Abstand voneinander) mit verschiedenen oder gleichen spektralen Signaturen markierten Objekt-(Sub-) Strukturen - im folgenden auch Meßstrukturen genannt - wird anhand der hochpräzisen Lokalisation unabhängiger (Kalibrier-)Targets mit entsprechend spektraler Signatur und mit bekannter Größe und räumlicher Anordnung, unter Berücksichtigung des wellenlängenabhängigen Abbildungs- und Lokalisationsverhaltens des jeweiligen optischen Systems durchgeführt, wobei die Kalibriermessung zwischen den (Kalibrier-) Targets und die Messung im biologischen Objekten unter gleichen System- und Randbedingungen stattfindet. Diese Kalibriertargets haben dieselbe oder eine höhere Multispektralität wie die zu messenden (Objekt-)Strukturen. Sie können direkt in den biologischen Objekten angeordnet sein, oder als separate Präparate auf einem Objekthalter (Objektträgerplättchen oder Objektträgerfaser/-kapillare oder Objektträgerflüssigkeit o.ä.) vorliegen oder Teil eines Objekthalters sein.

Zwei oder mehrere fluoreszierende Meßstrukturen in intakten, dreidimensionalen biologischen Objekten, deren Abstand und Ausdehnung kleiner als die Halbwertsbreite des Hauptmaximums der effektiven Punktbildfunktion ist, können aufgrund ihrer unterschiedlichen spektralen Signatur (Fluoreszenzabsorptionswellenlängen und/oder Fluoreszenzemissionswellenlängen und/oder Fluoreszenzemissionslebensdauern) diskriminiert werden, d.h. ihr Abstand untereinander kann bestimmt werden.

Die Abstandsmessung wird auf die Lokalisation der einzelnen Meßstrukturen reduziert und kann — nun auch in der optischen Fernfeldmikroskopie oder Flußfluorometrie — mit einer wesentlich höheren Genauigkeit als die Halbwertsbreite des Hauptmaximums der Punktbildfunktion durchgeführt werden. Die Lokalisation des Schwerpunkts der betreffenden Meßstrukturen wird auf die Maximalintensität ihres Fluoreszenzsignals angepaßt. D. h. aus dem gemessenen (beugungsbegrenzten) Signals (=Intensitätskurve) eines Fluoreszenzpunktes (=fluoreszierende Meßstruktur) wird — unter Berücksichtigung der Gesamtinformation aus Haupt- und Nebenmaxima — der Schwerpunkt (das Baryzentrum) des Signals bestimmt und damit der Ort der Meßstruktur. Bei fehlerfreiem optischen System und infolgedessen idealer Symmetrie der gemessenen Intensitätsverteilung (=Verlauf der Intensitätskurve) kolokalisiert der Schwerpunkt (das Baryzentrum) der Intensitätskurve innerhalb der Lokalisationsgenauigkeit mit dem Hauptmaximum (=Maximum 0. Ordnung des Beugungsbildes) der gemessenen Intensitätsverteilung.

Dieses neue Kalibrierverfahren erlaubt es mittels optischer Fernfeld-Mikroskopie wie z.B. der Wellenfeldmikroskopie (oder auch mittels Scanning-Flußfluorometrie,) geometrische Distanzen in biologischen Mikro-Objekten zu messen, wobei die zu bestimmenden Distanzen geringer sein können als die Halbwertsbreite des Hauptmaximums der effektiven Punktbildfunktion im Objekt. Da der Informationsgehalt der damit durchgeführten Distanzbestimmungen einer bei erhöhter Auflösung gewonnenen Distanzmessung entspricht, kann (und wird im folgenden) auch abkürzend von "Auflösungsäquivalent" gesprochen werden.

Die multispektrale Kalibrierung erlaubt es, in situ Messungen über das Abbildungsverhalten des Systems am konkreten biologischen Objekt durchzuführen. Bei Verwendung der Fluoreszenzlebensdauer als alleinigem Parametertyp und/oder bei Anregung der Fluorochrome mit derselben bzw. denselben Photonenenergie(n) entfällt aufgrund der Kalibrierung die in situ Korrektur des chromatischen Versatzes in der Objektebene. Für höchstauflösende Fernfeld-Mikroskoptypen wie z.B. das Wellenfeldmikroskop und bei Verwendung geeigneter Fluoreszenzmarker ermöglicht dieses Kalibrierverfahren dreidimensionale geometrische Distanzmessungen in biologischen Objekten bis hinunter zu molekularer Präzision (d.h. Auflösungsäquivalent besser 10 nm).

Zur Ermittlung von Ist- und Sollwerten, zu deren Vergleich und zur Bestimmung des Korrekturwerts/Kalibrierwerts werden vorzugsweise die folgenden Verfahrensschritte durchgeführt:

- ein oder mehrere Kalibriertargets B mit einem Abstand größer als die Halbwertsbreite des Hauptmaximum der effektiven Punktbildfunktion vom Schwerpunkt der N Meßstrukturen wird/werden mit einer beliebigen spektralen Signatur markiert,

- die Abstände $d_{ik}$ (i, k = 1... N, i≠k) der Schwerpunkte der spektral getrennten Beugungsfiguren der N Meßstrukturen und die Abstände $d_{iB}$ der N Meßstrukturen zum Kalibriertarget B werden gemessen, wobei automatisierte Verfahren der Bildanalyse angewendet werden,

- für eine Meßstruktur werden die Strecken $d_{ik}$ und $d_{iB}$ jeweils in der Ebene der schmalsten Punktbildfunktion sowie alle übrigen Distanzen gemessen werden, wozu das Objekt axialtomographisch jeweils um einen definierten Winkel $\phi_m$ gedreht wird,

- optische Aberrationen aus den Kalibrierungsmessungen werden korrigiert, und an die korrigierten gemessenen Abstände $d_{ik}(\phi_m)$ und $d_{iB}(\phi_m)$ wird jeweils eine Cosinusfunktion $A_{ik} \cos(\phi_m + \theta_{ik})$ bzw. $A_{iB} \cos(\phi_m + \theta_{iB})$ mit geeigneter Phasenverschiebung angepaßt,

- die Maxima $A_{ik}$ und $A_{iB}$ der Anpassungsfunktion von $d_{ik}$ bzw. $d_{iB}$ werden durch den Vergrößerungsfaktor dividiert und als euklidischer Abstand $D_{ik}$ bzw. $D_{iB}$ der N Meßstrukturen untereinander bzw. der Abstände der Meßstrukturen zum Bezugspunkt B bestimmt.

Für die Bestimmung der Maxima werden vorzugsweise zusätzlich die diesen entsprechenden Minima des Abstandes $z_{ik}$, $z_{iB}$ der zu der Ebene der $d_{ik}$, $d_{iB}$ orthogonalen Ebene herangezogen und analog ausgewertet.

Die Ermittlung aller Koordinaten der N Meßstrukturen und ihrer Relativkoordinaten zum Bezugspunkt B, d.h. die Ermittlung der Positionen $x_i$, $y_i$, $z_i$ und $x_k$, $y_k$, $z_k$ bzw. der Abstände $x_k-x_i$, $y_k-y_i$, $z_k-z_i$ und $x_B-x_i$, $y_B-y_i$, $z_B-z_i$, erfolgt erfindungsgemäß auf der Grundlage der mikroskopisch gemessenen 3D-Abstände $D_{ik}$ bzw. $D_{iB}$, vorzugsweise unter Verwednung des folgenden Gleichungssystems

$$D^2_{ik} = (x_k - x_i)^2 + (y_k - y_i)^2 + (z_k - z_i)^2$$

$$D^2_{iB} = (x_B - x_i)^2 + (y_B - y_i)^2 + (z_B - z_i)^2$$

$$D^2_{kB} = (x_B - x_i)^2 + (y_B - y_i)^2 + (z_B - z_i)^2$$

Zur Absicherung der ermittelten Meßergebnisse sollte die vorstehend beschriebene Vorgehensweise für mehrere Kalibriertargets B und die gleichen N Meßstrukturen durchgeführt werden.

Die Koordinaten und Abstände der N Meßstrukturen können anhand der Schwerpunkte ermittelt werden, die sich aus Schwerpunktmittelungen der Messungen zu allen Bezugspunkten ergeben.

Insbesondere für graphische Darstellungen werden die ermittelten Positionen $x_i$, $y_i$, $z_i$ und $x_B$, $y_B$, $z_B$ vorzugsweise mit einer Punktbildfunktion gefaltet, die eine Halbwertsbreite mit dem jeweils erreichten Auflösungsäquivalent besitzt.

Zur Fluorochrommarkierung von Meßstrukturen und Kalibriertargets werden vorzugsweise solche Fluorochrome verwendet, die im ultravioletten, sichtbaren und/oder infraroten Lichtwellenlängenbereich angeregt werden können und die im ultravioletten, sichtbaren und/oder infraroten Lichtwellenlängenbereich emittieren.

Als Kalibriertargets können entweder biologische Kalibriertargets oder nicht-biologische bzw. synthetische Kalibriertargets eingesetzt werden.

Bei den biologischen Kalibriertargets handelt es sich um markierte Regionen des biologischen Objekts mit bekannter Distanz voneinander. Die Markierung(en) der betreffenden Region(en) kann(können) beispielsweise mit geeigneten biochemischen Sonden durchgeführt werden. Die Verwendung solcher biologischer Kalibriertargets hat gegenüber der Verwendung synthetischer Kalibriertargets, beispielsweise Kalibrierkügelchen, den praktischen Vorteil, daß bei der Kalibrierung neben den optischen Randbedingungen des Objektes zusätzlich präparativ bedingte Randeffekte in die Kalibrierung einfließen, wie z.B. das Verhältnis aus tatsächlichen Fluoreszenzsignal zu unspezifischem Hintergrund (das durch automatische Bildanalysealgorithmen bestimmt wird).

Als nicht-biologische bzw. synthetische Kalibriertargets eignen sich ganz besonders Mikrokügelchen, die die gleiche oder eine höhere multispektrale Signatur als die zu ortenden Meßstrukturen aufweisen. Sie werden wie die biologischen Objekte behandelt. Solche Kalibriertargets sind vorzugsweise auf Objekthaltern in definierter Raumanordnung fixiert. Die Fixierung kann bereits bei der Fabrikation der betreffenden Objektträger geschehen, was insbesondere für die Routinebenutzung von Vorteil ist.

Zur Behebung des bei allen bekannten Fernfeldmikroskopieverfahren bestehenden Problems, daß die Breite des Hauptmaximums der Punktbildfunktion und damit die Auflösungsgrenze von der relativen Lage im Raum abhängt, d.h. z.B. senkrecht zur optischen Achse (= lateral) schmäler ist als in Richtung der optischen Achse (= axial), kann das genannte Kalibrierverfahren sehr gut mit den im Stand der Technik bekannten sog. Mikroaxialtomographieverfahren kombiniert werden. Bei diesen Mikroaxialtomographieverfahren werden die (biologischen) Objekte in Kapillaren oder auf Glasfasern angeordnet und im bzw. unter dem Mikroskop definiert um eine Achse, die normalerweise senkrecht zur optischen Achse des Mikroskops steht, gedreht werden, wobei Abstandsmessungen in derjenigen Richtung durchgeführt werden, die die schmalste Halbwertsbreite der effektiven Punktbildfunktion besitzt.

Ein Fernfeld-Lichtmikroskopieverfähren, das sich für die Detektion und Abbildung von insbesondere sehr kleinen, durch Fluoreszenzmarker kenntlich gemachte Substrukturen in biologischen Objekten besonders eignet, weil es gegenüber den bekannten Epifluoreszenzmikroskopieverfahren oder der konfokalen Laser-Scanning-Mikroskopie den Vorteil aufweist, daß es auch in axialer Richtung - senkrecht zu den Wellienfronten - eine Tiefendiskriminierung und damit wesentlich verbesserte Auflösung ermöglicht (ihre Dimension kann bei hoher Numerischer Apertur wesentlich geringer als die Wellenlänge des zur Anregung verwendeten Lichtes sein), ist das Wellenfeldmikroskopieverfahren.

Bei der Wellenfeldmikroskopie, wie sie z.B. in der US-Patentschrift 4,621,911 beschrieben ist, werden fluoreszierende bzw. lumineszierende Präparate im optischen Mikroskop mit einen stehenden Wellenfeld beleuchtet (Standing Wave Field Fluorescence Microscopy, SWFM). Ein stehendes Wellenfeld entsteht (nur) dort, wo Licht kohärenzfähig überlagert wird. Die Präparate werden in einer Zone äquidistanter ebener Wellenfronten angeordnet und zur Fluoreszenz oder Phosphoreszenz angeregt. Der Abstand der Wellenfronten und ihre Phase können (insbesondere zur Bilderzeugung) variiert werden. Aus einzelnen optischen Schnitten kann durch Computer-Bildverarbeitung die dreidimensionale Verteilung der fluoreszenten bzw. lumineszenten Objektpunkte rekonstruiert werden.

Die ebenen Wellenfronten sind senkrecht zur optischen Achse des detektierenden Objektives angeordnet und werden durch kohärente Überlagerung zweier Laserstrahlen unter einem definierten Winkel $\theta$ zur optischen Achse des Mikroskopsystems erzeugt, wobei der Winkel $\theta$ den Abstand der Wellenfronten untereinander bestimmt — bei gegebener Wellenlänge und Brechungsindex. An Stelle von zwei sich kreuzenden Laserstrahlen kann das Wellenfeld auch dadurch erzeugt werden, daß ein Laserstrahl nach geeigneter Reflexion unter einem bestimmten Winkel (z.B. mit einem Spiegel) mit sich selbst zur Interferenz gebracht wird. Die ebenen Wellenfronten zeichnen sich dadurch aus, daß der Intensitätsverlauf in Richtung senkrecht zu den Wellenfronten (co)sinusförmig ist. Die Fluoreszenz bzw. Lumineszenz wird entweder durch entsprechende optische Filter spektral diskriminiert und in verschiedenen Strahlengängen geführt oder konfokal detektiert. Die erzielbare Auflösung, d.h. . die kleinste noch meßbare Distanz zwischen zwei punktförmigen Objektstrukturen, die mit Fluorochromen gleicher spektraler Signatur markiert sind, ist entweder durch das Abbe-Kriterium (= das Maximum 0. Ordnung des Beugungsbildes eines Punktobjektes ist im 1, Minimum des Beugungsbildes eines zweiten Punktobjektes lokalisiert) oder durch die Halbwertsbreite des Hauptmaximums der effektiven Punktbildfunktion gegeben. Sie hängt von der jeweiligen Wellenlänge, der Numerischen Apertur des verwendeten Objektivs, sowie von den lokalen Brechzahlen der Objekte, des Einbettungsmediums, der eventuell verwendeten Deckgläser und der eventuell eingesetzter Immersionsflüssigkeiten ab.

Die bekannten Wellenfeldmikroskope sind im Prinzip wie folgt aufgebaut: Sie umfassen

(I) ein Beleuchtungs- bzw. Anregungssystem, bestehend aus

wenigstens einer reellen und einer virtuellen Beleuchtungsquelle und wenigstens einem Objektiv, die derart zueinander angeordnet sind, daß sie zur Erzeugung eines eindimensionalen, sinusförmigen, stehenden Wellenfeldes geeignet sind,

(II) einen Objektraum, mit

Halte und Manövriervorrichtungen für das Objekt, und

(III) ein Detektionssystem, bestehend aus

wenigstens einem Objektiv,

wenigstens einem Okular

und wenigstens einem Detektor, wobei es sich häufig um eine Kamera, insbesondere eine CCD-Kamera handelt, die so positioniert ist, daß der CCD-Chip in der Zwischenbildebene liegt.

Ein Nachteil dieses Wellenfeldmikroskops nach dem Stand der Technik, im folgenden "eindimensionales Wellenfeldmikroskop" ("SWFM") genannt, bzw. der damit durchführbaren Wellenfeldmikroskopieverfahren besteht darin, daß das periodisch erzeugte Wellenfeld (bei epifluoreszenter Detektion in Verbindung mit Verfahren des Optical Sectioning) zu einer Mehrdeutigkeit in der Aufnahme bzw. Abbildung einer Objektstruktur führt, deren Ausdehnung in Richtung senkrecht zu den Wellenfronten wesentlich größer als $\lambda/2n$ ist ($\lambda$=Wellenlänge der Anregung, n= effektiver Brechungsindex). Diese Mehrdeutigkeit erschwert zunächst eine effektive Nutzung der durch das Interferenzmuster erzielten Auflösungsverbesserung.

Zur Durchführung von Distanzmessungen und anderen Untersuchungen der räumlichen Verhältnisse von dreidimensionalen Objekten können die bekannten Fernfeldmikroskopieverfahren einschließlich der eindimensionalen Wellenfeldmikroskopie mit Axialtomographie kombiniert werden. Hierzu werden die zu untersuchenden biologischen Objekte, ggf. nach Ausrüstung mit Kalibriertargets, in oder auf einer Mikrokapillare oder Glasfaser als Objekthalter bzw. Objektträger präpariert. Die Kapillare/Faser hat einen exakt definierten Durchmesser, wobei verschiedene Durchmesser möglich sind. Zur Festlegung dieser Kapillare/Faser auf dem Mikroskoptisch dient eine spezielle Halterung vorgeschlagen, die aus einem starren, vorzugsweise dorsiventral abgeplatteten Rahmen besteht, an bzw. auf dem wenigstens eine Lagerbuchse montiert ist, in der eine Mikrokapillare oder Glasfaser um ihre Längsachse rotierfähig (vorzugsweise mit der Rotationsachse senkrecht zur optischen Achse des Mikroskops) gelagert werden kann. (Die Lagerbuchse(n) sollten so angeordnet sein, daß die Rotationsachse der Kapillare/Faser senkrecht zur optischen Achse des Mikroskops verläuft.) Die Drehung der Untersuchungsobjekte in oder an der Kapillare/Faser erfolgt durch Drehung der Kapillare/Faser direkt, vorzugsweise vermittels eines Drehmotors.

Der vorliegenden Erfindung liegt die Aufgabe zugrunde, ein Wellenfeldmikroskop der bekannten Art derart weiter zu bilden, daß es zur Erzeugung von ebenen Wellenfeldern in mehr als einer Dimension bei hoher Variabilität der Abstände der Interferenzmaxima geeignet ist, und das vorgenannte Kalibrierverfahren derart weiter zu entwickeln, daß es in Kombination mit einem solchen Wellenfeldmikroskop einsetzbar ist. Darüber hinaus soll ein Verfahren zur wellenfeldmikroskopischen DNA-Sequenzierung geschaffen werden.

Eine Lösung dieser Aufgabe besteht einerseits in der Bereitstellung der nachstehend beschriebenen sog. "mehrdimensionalen Wellenfeldmikroskope", andererseits in der Bereitstellung des ebenfalls nachstehend beschriebenen, an die Anwendung eines mehrdimensionalen Wellenfeldmikroskops angepaßten Kalibrierverfahrens. Darüber hinaus wird ein Verfahren zur "Fluoreszenz-DNA-Sequenzierung" bereit gestellt.

Bei dem einen erfindungsgemäßen "mehrdimensionalen Wellenfeldmikroskop" (Typ 1) handelt es sich um ein Wellenfeldmikroskop der eingangs genannten Art, das durch die nachfolgend aufgelisteten Merkmale gekennzeichnet ist:

(1) Das Beleuchtungs- bzw. Anregungssystem umfaßt in zwei oder allen drei Raumrichtungen wenigstens eine reelle oder virtuelle Beleuchtungsquelle für kohärenzfähige Lichtstrahlen und wenigstens einen Reflektor oder Strahlteiler zur Auskopplung von Teilstrahlen oder eine weitere Beleuchtungsquelle für kohärenzfähige Lichtstrahlen, denen jeweils wenigstens ein Objektiv zugeordnet ist, und die jeweils zur Erzeugung von Lichtwellenzügen geeignet sind, wobei die Lichtwellenzüge der einen Beleuchtungsquelle antiparallel oder in variabel einstellbaren Winkeln zu den Lichtwellenzügen des Reflektors bzw. der anderen Beleuchtungsquelle ausgerichteten sind, und zwar derart, daß die von der einen Beleuchtungsquelle ausgesendeten Lichtwellenzüge mit denen des Reflektors bzw. der anderen Beleuchtungsquelle zu einem stehenden Wellenfeld mit ebenen Wellenfronten interferieren.

(2) Das Detektionssystem umfaßt wenigstens ein zur epifluoreszenten Detektion geeignetes und/oder wenigstens ein zur rasternden Punktdetektion geeignetes Detektionsobjektiv vorzugsweise hoher numerischer Apertur, welches mit seiner optischen Achse senkrecht zu den Wellenfronten eines der interferierenden Wellenfelder angeordnet ist und welches mit einem Objektiv des Anregungssystems identisch sein kann. Dem zur epifluoreszenten Detektion geeigneten Detektionsobjektiv ist ein flächiger (zweidimensionaler) Detektor, z.B. eine Kamera vorgeordnet, während dem zur rasternden Punktdetektion geeigneten Detektionsobjektiv wenigstens eine feststehende konfokale Detektionsringblende und/oder -lochblende und/oder wenigstens ein feststehender Detektionsschlitz vorgeordnet und ein Punktdetektor, insbesondere ein Photomultiplier, eine Photodiode oder eine Diodenarray nachgeordnet ist.

Unter "hoher" numerischer Apertur ist hier eine numerische Apertur ≥ 1 und unter "niedriger" numerischer Apertur eine numerische Apertur < 1 zu verstehen.

Bei einer besonders bevorzugten Ausführungsform dieses Wellenfeldmikroskops (Typ I) ist in wenigstens einer Raumrichtung einem Objektiv hoher numerischer Apertur ein Objektiv niedriger numerischer Apertur oder ein Reflektor zugeordnet, und in einer oder beiden anderen Raumrichtung(en) sind entweder zwei Objektive niedriger numerischer Apertur oder ein Objektiv niedriger numerischer Apertur und ein Reflektor einander zugeordnet.

Bei dem anderen erfindungsgemäßen "mehrdimensionalen Wellenfeldmikroskop" (Typ II) handelt es sich um ein Wellenfeldmikroskop der eingangs genannten Art, das durch die folgenden Merkmale charakterisiert ist:

(1) Das Beleuchtungs- bzw. Anregungssystem umfaßt in wenigstens einer der drei Raumrichtungen wenigstens eine reelle oder virtuelle Beleuchtungsquelle für kohärenzfähige Lichtstrahlen und wenigstens einen Strahlteiler zur Auskopplung von wenigstens einem Teilstrahl, denen ein gemeinsames Objektiv zugeordnet ist, in das die Lichttrahlen bzw. Lichtwellenzüge der Beleuchtungsquelle(n) und des/der Strahlteiler(s) derart eingekoppelt werden können, daß sie auf der hinteren (dem Objektraum abgewandten) Fokalebene zwei voneinander beabstandete Fokuspunkte erzeugen und in dem Raum zwischen den beiden Fokalebenen in variabel einstellbarem Winkel zueinander verlaufen und zu einem eindimensionalen stehenden Wellenfeld interferieren.

(2) Das Detektionssystem umfaßt wenigstens ein zur epifluoreszenten Detektion geeignetes und/oder wenigstens ein zur rasternden Punktdetektion geeignetes Detektionsobjektiv vorzugsweise hoher numerischer Apertur, das auch mit einem Objektiv des Anregungssystems identisch sein kann. Dem zur epifluoreszenten Detektion geeigneten Detektionsobjektiv ist ein flächiger (zweidimensionaler) Detektor, z.B. eine Kamera vorgeordnet, während dem zur rasternden Punktdetektion geeigneten Detektionsobjektiv wenigstens eine feststehende konfokale Detektionsringblende und/oder -lochblende und/oder wenigstens ein feststehender Detektionsschlitz vorgeordnet und ein Punktdetektor, insbesondere ein Photomultiplier, eine Photodiode oder eine Diodenarray nachgeordnet ist,

Bei einer bevorzugten Ausführungsform dieses Wellenfeldmikroskops (Typ II) weist das Beleuchtungs- bzw. Anregungssystem in derselben oder einer oder beiden anderen Raumrichtung(en) jeweils wenigstens eine weitere reelle oder virtuelle Beleuchtungsquelle für kohärenzfähige Lichtstrahlen und/oder wenigstens einen Strahlteiler zur Auskopplung von wenigstens einem Teilstrahl auf; dem/denen jeweils ein weiteres Objektiv zugeordnet ist, durch das der/die Lichtstrahlen (Lichtwellenzüge) in den Objektraum gelenkt und derart ausgerichtet sind, daß sie mit den Lichtstrahlen aus derselben oder aus der /den beiden anderen Raumrichtung(en) bzw. dem von diesen gebildeten ein- oder zweidimensionalen Wellenfeld zu einem zwei- bzw. dreidimensionalen Wellenfeld interferieren.

Eine weitere, sehr vorteilhaften Weiterbildung sämtlicher vorstehend genannten erfindungsgemäßen Wellenfeldmikroskope (Typ I und Typ II) zeichnet sich dadurch aus, daß der Objektraum eine Objekthalterung umfaßt, in bzw. an der das Objekt mit den Meßstrukturen und/oder gegebenenfalls den/die Kalibriertarget(s) um eine oder zwei orthogonal zueinander verlaufende Achsen drehbar in dem Wellenfeld gelagert ist, wobei für wenigstens eine Achse eine Drehbarkeit um 360-Grad (2π) bevorzugt ist.

Mit diesen erfindungsgemäßen mehrdimensionalen Wellenfeldmikroskopen Typ I und Typ II ist es möglich, eine zeitlich sequentielle und/oder simultane Detektion mehrerer Objektebenen durch eine, zwei und/oder drei Objektive (bzw. zu deren orthogonalen Achsen) durchzuführen. Präzisionsdistanzmessungen von Punktobjekten gleicher oder verschiedener spektraler Signatur, deren Abstände kleiner als die Halbwertsbreiten der Hauptmaxima der effektiven Punktbildfunktionen sind, können in (allen) Raumrichtungen vorgenommen werden.

Die feststehende(n) konfokale(n) Detektionsringblende(n), -lochblende und/oder der/die feststehende(n) Detektionsschlitz(e) in Kombination mit wenigstens einem geeigneten Lichtintensitätsdetektor bietet/bieten die vorteilhafte Möglichkeit, daß das Objekt in x-, y- und z-Richtung durch das Wellenfeld gerastert werden kann (Objekt- oder Stage-scanning).

Das erfindungsgemäße Wellenfeldmikroskop Typ II und die damit durchführbaren Wellenfeldmikroskopie hat — insbesondere gegenüber der bekannten eindimensionalen Wellenfeldmikroskopie — überdies den Vorteil, daß sowohl die laterale Auflösung (d.h. senkrecht zur optischen Achse) als auch die axiale Auflösung wesentlich verbessert ist. Es ist erstmals eine Diskriminierung von flächenhaften Objekten in axialer Richtung ohne den Einsatz von konfokalen Systemen möglich. Außerdem besteht die vorteilhafte Möglichkeit, daß man das Objekt während der Untersuchung bzw. zur Aufnahme / Datenregistrierung in lateraler Richtung verschieben kann. Mit Hilfe von Bildverarbeitungs- und Rekonstruktionsverfahren läßt sich dann aus den somit erhaltenen Mehrfachaufnahmen eine höhere laterale Auflösung gewinnen. Der erfindungsgemäße Aufbau Typ II mit einem Objektiv eignet sich außerdem auch zur Erzeugung eines eindimensionalen Wellenfeldes senkrecht zur optischen Achse eines Epifluoreszenzmikroskops und damit zur lateralen Auflösungsverbesserung desselben.

Bei einer weiteren Ausführungsvariante dieser "Mehrdimensionalen Wellenfeldmikroskope" (Typ I und/oder Typ II) sind die das mehrdimensionale Wellenfeld erzeugende(n) Beleuchtungsquelle(n) und/oder der/die Reflektor(en) und/oder der/die Strahlteiler und/oder das/die Objektiv(e) und damit das mehrdimensionale Wellenfeld um eine oder zwei orthogonal zueinander verlaufende Achsen drehbar angeordnet bzw. montiert.

Zur Abbildung der lateralen Objektbereiche eines im zwei- oder dreidimensionalen Wellenfeld feststehenden Objekts auf die Detektorringblende, -lochblende bzw. den Detektorschlitz kann jedes erfindungsgemäße Wellenfeldmikroskop (Typ I und/oder Typ II) mit einem Scannerspiegel ausgerüstet sein, der derart angeordnet ist, daß er die betreffenden lateralen Objektbereiche mit der gewünschten, meist maximalen, Fluoreszenzintensität abbildet.

Bei einer besonders vorteilhaften Weiterbildung der erfindungsgemäßen mehrdimensionalen Wellenfeldmikroskope (Typ 1 und/oder Typ II), die für Zwei- oder Mehr-PhotonenFluoreszenzanregungen geeignet ist, den sog. "Wellenfeldmikroskopen mit kombinierter Mehr-Photonen-Fluoreszenzanregung", umfaßt das jeweilige Beleuchtungssystem in wenigstens einer der drei Raumrichtungen eine reelle Beleuchtungsquellen für die Zwei- oder Mehrphotonenanregung und in einer oder beiden anderen Raumrichtung(en) eine reelle und/oder virtuelle Beleuchtungsquelle für die Zwei- oder Mehrphotonenanregung. Die damit erzeugten stehenden Wellenfelder ($WF_1$, $WF_2$, . . ., $WF_i$) weisen voneinander verschiedene Wellenlängen ($\lambda_1$, $\lambda_2$ ..., $\lambda_i$) auf, und die Distanzen ($d_1$, $d_2$, ..., $d_i$) zwischen ihren jeweiligen Wellenmaxima bzw. Wellenminima betragen $d_1 = \lambda_1/2n \cos\theta_1$ bzw. $d_2 = \lambda_2/2n \cos\theta_2$ bzw. $d_i = \lambda_i/2n \cos\theta_i$ (mit: n = Brechungsindex im Objektraum, $\theta_1$, $\theta_2$, .... $\theta_i$ = Kreuzungswinkel des Lichtwellenzugs der Wellenlänge $\lambda_1$, $\lambda_2$ ...,$\lambda_i$ mit der optischen Achse). Diese Wellenfelder $WF_1$,$WF_2$ ... $W_i$ sind erfindungsgemäß derart zueinander ausgerichtet, daß sich mindestens ein Maximum zweier oder aller stehenden Wellen an derselben Stelle, nämlich dem Ort einer Mehrphototonenanregung, befindet.

Geeignete Beleuchtungsquellen für die Zwei- oder Mehrphotonenanregung sind im Stand der Technik bekannt und z.B. in der Druckschrift von W.Denk, J.H. Strickler, W.W. Webb, "Two-Photon Laser Scanning Fluorescence Microscopy", Science, Vol. 248, pp.73-76 (6. April 1990) beschrieben, auf deren Inhalt hier ausdrücklich Bezug genommen wird. Diese Beleuchtungsquellen produzieren entweder Photonen unterschiedlicher Wellenlängen oder kohärente Photonen gleicher Wellenlänge.

Ein besonderer Vorteil der Kombination aus Ein- und Zwei- oder Mehrphotonenanregung besteht in der gleichzeitigen Anregung von Fluoreszenzmarkern verschiedener spektraler Signatur. Hierdurch können Fehler der Distanzmessung aufgrund der chromatischen Aberration im Objekt eliminiert werden. Bei der Zwei-Photonen-Anregung wird ein Fluorochrommolekül dann angeregt, wenn zwei Photonen simultan die Energie für die Anregung eines Moleküls liefern. Dabei können die zwei an der Anregung des Moleküls beteiligten Photonen dieselbe oder unterschiedliche Wellenlängen bzw. Energien besitzen. Für eine koinzidente Anregung mit unterschiedlichen Wellenlängen ($\lambda_1$, $\lambda_2$) müssen bei der sog. "Zwei-Photonen-Wellenfeldmikroskopie" in jeder jeweiligen Raumrichtung jeweils zwei Wellenfelder mit den Wellenlängen $\lambda_1$ und $\lambda_2$ installiert sein. Die Wellenmaxima bzw. Wellenminima der beiden stehenden Wellenfelder (WF1, WF2) haben dabei die Distanzen $d_1 = \lambda_1/2n \cos\theta_1$ bzw. $d_2 = \lambda_2/2n \cos\theta_2$ (wobei gilt: n = Brechungsindex im Objektraum, $\theta_1$, $\theta_2$ = Kreuzungswinkel des Laserstrahls der Wellenlänge $\lambda_1$, $\lambda_2$ mit der optischen Achse). Da im allgemeinen die Distanzen $d_1$ und $d_2$ verschieden sind, werden die beiden Wellenfelder pro Raumrichtung so ausgerichtet, daß ein Hauptmaximum beider stehender Wellen sich an derselben Stelle befindet. Mehrphotonen-Effekte können dann nur dort auftreten, wo beide Wellenfelder sich überlagern. Damit werden nur noch einzelne "'Streifen'" des Wellenfeldes einer Raumrichtung zur Mehrphotonenanregung genutzt. Vieldeutigkeiten bei Objekten der Dimension größer d treten erst bei Dimensionen mit der Bedingung $k_1 d_1 = k_2 d_2$ ($k_1$, $k_2$ sind ganze Zahlen) auf. Durch eine Mehrphotonenanregung der fluoreszenzmarkierten Meßstrukturen und Kalibriertargets wird somit eine Eindeutigkeit der dreidimensionalen Abbildung erreicht. Die Verwendung von Zwei- oder Mehrphotonenfluoreszenzanregungsverfahren ermöglicht ein schnelleres Abscannen bzw. Abrastern des Objekts, d.h. der Objektpunkte, -linien und -ebenen und damit eine bessere Abbildungsqualität insbesondere auch bei sich bewegenden Objekten.

Eine ebenfalls sehr vorteilhafte Weiterbildung des erfindungsgemäßen mehrdimensionalen Wellenfeldmikroskops zeichnet sich dadurch aus, daß eine Anordnung aus Lichtquelle, Objektiv und einem elektrisch leitenden Spiegel, die zur Erzeugung eines eindimensionalen, elektrischen Wellenfeldes geeignet ist, relativ zur Objektträgerhalterung vorgesehen ist, und zwar derart, daß die in dem Objekt befindlichen Meßstrukturen und/oder Kalibriertargets im Bedarfsfall (z.B. wenn sich diese an bzw. in Molekülketten befinden) durch Anlegen des elektrischen Feldes — vor oder während des mikroskopischen Meßvorgangs — ausgerichtet werden können. (Die auf diese Weise räumlich ausgerichteten Molekülketten können dann anschließend noch mit immobilisierenden Substanzen fixiert werden.) Diese Variante des erfindungsgemäßen Wellenfeldmikroskops ist vor allem für die wellenmikroskopische DNA-Sequenzierung geeignet, wobei das eindimensionale elektrische Wellenfeld zur Kalibrierung bei der DNA-Sequenzierung dient.

Zur Detektion des Lumineszenzlichtes wird vorzugsweise eine CCD-Kamera(s) in bekannter Weise eingesetzt. Diese kann hinter der Detektorringblende oder -lochblende oder dem Detektorschlitz sitzen. Anstelle der CCD-Kamera bzw. des CCD-Chips kann das erfindungsgemäße mehrdimensionale Wellenfeldmikroskop aber auch mit einer elektronischen Bildaufnahmevorrichtung ausgerüstet sein, wie sie beispielsweise von Konfokalen Laser-Scan-Miksroskopen (CLSM) im Stand der Technik bekannt ist. Erfindungsgemäß kann prinzipiell jedes lichtempfindliche Detektionsgeräte, insbesondere Photodiode(n), Photomultiplier, CCD-Kameras/ -Chips, CCD-Arrays, Avalanche Dioden, (Avalanche) Dioden Arrays, zweidimensionale (Avalanche) Dioden Matrizes hinter der hinter der Detektorringblende oder -lochblende oder dem Detektorschlitz angeordnet sein, um die Fluoreszenz zu detektieren und zu dokumentieren, wobei auch Fluoreszenzlebensdauermessungen vorgenommen werden können.

Bei dem erfindungsgemäßen Kalibrierverfahren handelt es sich um ein Kalibrierverfahren der vorstehend genannten Art, das durch die folgenden Maßnahmen gekennzeichnet ist:

(1) Das biologische Objekt mit den fluorochrommarkierten Meßstrukturen und/oder den/die fluorochrommarkierten Kalibriertargets wird sequentiell oder simultan mit einzelnen (separaten), in zwei oder drei Raumrichtungen orthogonal zueinander verlaufenden, stehenden und zu einem zwei- oder dreidimensionalen Wellenfeld miteinander interferierenden Wellenfeldern beleuchtet, wobei die Fluorochrome zur Fluoreszenzemission angeregt werden.

(2) Zur Detektion der Fluoreszenzintensität wird/werden eine Kamera und/oder eine oder mehrere zweidimensionale Anordnung(en) aus Einzeldetektoren mit jeweils kreis-, ring- oder schlitzförmiger Blende oder eine Anordnung von mehreren kreis-, ring- oder schlitzförmigen Blenden verwendet

(3) Entweder das Objekt mit den Meßstrukturen und/oder die/den Kalibriertargets oder das ein- bzw. zweidimensionale Wellenfeld oder beides wird während des Meßvorgangs schrittweise um eine oder zwei orthogonal zueinander verlaufende Achsen gedreht, wobei die fluorochrommarkierten Meßstrukturen und/oder Kalibriertargets sequentiell oder simultan mit einem oder zwei einzelnen, orthogonal zueinander stehenden Wellenfeldern beleuchtet werden.

Bei der simultanen Beleuchtung wird das Mikroobjekt mit den Meßstrukturen und den bzw. die Kalibriertargets starr oder um eine Achse drehbar gelagen. Zwei oder drei orthogonal zueinander verlaufende, stehende, ebene Wellenfelder werden zur Interferenz gebracht und beleuchten das Mikroobjekt simultan. Bei zwei Wellenfeldern entstehen Ebenen mit einem zweidimensionalen symmetrischen Gitter aus Punkten maximaler und minimaler Intensität. Bei der Verwendung von drei Wellenfeldern entsteht ein dreidimensionales Raumgitter aus symmetrisch regelmäßig angeordneten Punkten maximaler und minimaler Intensität. Zwischen den Intensitätsmaxima und -minima liegt ein kontinuierlicher Intensitätsverlauf vor.

Bei der sequentiellen Beleuchtung wird das Mikroobjekt im Wellenfeld um zwei Achsen gedreht. Während oder nach der Detektion kann die Lage des Wellenfeldes relativ zum Objekt verändert werden.

Bei Verwendung geeigneter Fluoreszenzmarker erlaubt die Erfindung somit, dreidimensionale (3D), geometrische Distanzmessungen zwischen Fluoreszenztargets gleicher bzw. verschiedener spektraler Signatur mit molekularer Präzision, d.h. mit einem 3D Auflösungsäquivalent bis zu besser als 10 nm und mit einer 3D Lokalisationsgenauigkeit bis zu besser als 1 nm. Im Gegensatz zur Elektronenmikroskopie bzw. zur optischen und nichtoptischen Nahfeldmikroskopie bleibt die dreidimensionale Struktur des zu untersuchenden Objektes intakt, da auf mechanische Schnitte verzichtet wird. Damit können in dreidimensional konservierten Mikroobjekten 3D-Distanzmessungen in einem Bereich kleiner als die Halbwertsbreite der Hauptmaxima der effektiven Punktbildfunktionen vorgenommen werden. Insbesondere eröffnet das Verfahren die Möglichkeit, dreidimensionale Distanzmessungen auch unter vitalen Bedingungen des biologischen Objektes durchzuführen. Bei der DNA-Sequenzierung kann die Herstellung von Gelen und die elektrophoretische Auftrennung der DNA-Stücke entfallen. Ebenso kann auf eine Autoradiographie verzichtet werden, da keine radioaktive Markierung durchgeführt wird. Und auch lange DNA-Sequenzen (z.B. > 1 kbp) können ohne weiteres analysiert werden.

Diese erfindungsgemäße Verfahrensvariante erlaubt außerdem eine wesentlich verbesserte Bestimmung auch morphologischer Größen (z.B. Volumen, Oberflächen), sofern die multispektralen Fluoreszenzmarker in geeigneter Weise z.B. auf der Oberfläche des Objektes, verteilt werden. Beispielsweise kann auf diese Weise das Volumen eines sphärischen Mikroobjektes mit einem Radius von einigen 100 nm erheblich besser bestimmt werden als das mit herkömmlichen mit morphologischen Segmentierungstechniken, wie z.B. Cavalieri- und Voronoi Verfahren, oder auch Methoden des Volumenkonservierenden Gradual Thresholding möglich ist.

Mit dem bzw. den erfindungsgemäßen mehrdimensionalen Wellenfeldmikroskopen (Typ I und/oder Typ II) und dem erfindungsgemäßen Kalibrierverfahren ist es möglich eine mikroskopische DNA-Sequenzierung durchzuführen. Hierfür wird erfindungsgemäß das nachfolgen beschriebene "Wellenfeldmikroskopieverfahren zur DNA-Sequenzierung" vorgeschlagen:

(1) Es werden alle komplementären Subsequenzen der zu analysierenden DNA-Sequenz derart hergestellt, daß alle Subsequenzen am selben Nukleotid der zu analysierenden Sequenz beginnen.

(2) Die zu analysierenden Bruchstücke werden alle am 3'-Ende mit einem Bezugsfluorochrommarker α und am 5'-Ende und/oder an definierten Zwischenstellen mit einem Fluorochrommarker a, g, c,oder t — je nachdem ob das Nukleotid die Base Adenin (Marker a), (Guanin (Marker g), Cytosin (Marker c) oder Thymin (Marker t) trägt — markiert, wobei die Fluorochrommarker a, g, c, t und α verschiedene spektrale Signatur aufweisen, und jeweils ein oder mehrere Fluorochrommoleküle enthalten.

(3) Die markierten DNA-Subsequenzen werden derart auf Träger fixiert, daß sie als lineare Sequenz vorliegen, und in ein ein- oder mehrdimensionales Wellenfeldmikroskop eingebracht.

(4) Die linearen DNA-Subsequenzen werden so zu den stehenden Wellenfronten orientiert, daß eine exakte Abstandsmessung (Genauigkeit $\leq 1 \cdot 10^{-10}$ m) zwischen α und a bzw. g, c oder t — nach Bestimmung der Intensitätsbaryzentren und Kalibrierung der Abbildungseingenschaften — durchgeführt werden kann, indem

(5) die Signale der Fluorochrommarker schrittweise, spektral getrennt voneinander registriert werden, und

(6) aus den Abständen der fluoreszenten Markern und ihrer spektralen Signatur die DNA-Basensequenz des zu analysierenden DNA-Stückes bestimmt wird.

Mit diesem im Stand der Technik völlig neuartigen Verfahren können DNA-Fragmente in ihrer Länge nukleotidgenau vermessen werden, und auch ihre Basensequenz kann exakt bestimmt werden. Gelelektrophorese und nachfolgende Bandenauswertung kann entfallen.

**Ausführungs- und Vergleichsbeispiele zur näheren Erläuterung der Erfindung:**

**BEISPIEL 1: Aufbau eines mehrdimensionalen Wellenfeldmikroskops Typ I mit drehbar gelagertem Objekt**

Die Basis stellt ein herkömmliches "eindimensionale" Wellenfeldmikroskop dar, das z.B. mit zwei sich gegenüber liegenden Objektiven hoher Numerischer Apertur oder mit einem Objektiv hoher gegenüber einem Objektiv niedriger numerischer Apertur oder mit einem Objektiv für zwei interferierende Laserstrahlen aufgebaut wird. Durch die Objektive werden zwei Teilstrahlen eines Lasers so zur Interferenz gebracht, daß ein eindimensionales stehendes Wellenfeld entsteht. Über ein oder zwei Objektiv(e) hoher Numerischer Apertur wird die Fluoreszenz detektiert. Jeweils in einer oder in beiden orthogonalen Richtungen zur optischen Achse des Detektionsobjektivs werden jeweils zwei weitere Teilstrahlen des Lasers über Objektive niedrigerer Numerischer Apertur und/oder Fokussierlinsensysteme in geeignetem Abstand eingekoppelt und so miteinander und mit dem eindimensionalen stehenden Wellenfeld zur Interferenz gebracht, daß ein zwei- oder dreidimensionales symmetrisches Intensitätsmuster aus Intensitätsmaxima und -minima entsteht.

In dieses "mehrdimensionale" Wellenfeldmikroskop wird zur Objektlagerung ein Mikroaxialtomograph eingebaut.

Bei der Axialtomographie wird anstelle des Glas-Objektträgers eine Mikrokapillare oder eine Glasfasern eingesetzt, die drehbar gelagert ist und das (biologische) Objekt in sich aufnimmt (Kapillare) oder an dem das (biologische) Objekt geeignet aufgebracht ist (Kapillare/Faser). Manuell oder mit einem computergesteuerten Schrittmotor kann die Kapillare/Faser, die üblicherweise senkrecht zur optischen Achse des Detektionsobjektivs angeordnet wird, um die Faserachse um einen definierten Winkel gedreht werden. Eine Drehung um einen Winkel von 360 Grad ($2\pi$) ist möglich. Der Trägerhalter für die Kapillare/Faser ist auf einem Halbkreis drehbar gelagert. Die Drehachse verläuft dabei senkrecht zur optischen Achse des Detektionsobjektivs.

Die Detektion der räumlichen Anordnung der Mikrotargets und ihrer Distanz erfolgt mittels des erfindungsgemäßen Kalibrierverfahrens und digitaler Bildanalyse. Mehrdeutigkeiten von Intensitätsverläufen, d.h. z.B. Intensitätshaupt- und -nebenmaxima von fluoreszenten "Punkt"-targets, können mit Hilfe geeigneter Computeralgorithmen statistisch ausgewertet werden und damit zu einer Erhöhung der Lokalisationspräzision beitragen. Bei ausgedehnten Objekten können Mehrdeutigkeiten durch Zwei- oder Mehr-Photonen Anregung mit Photonen verschiedener Wellenlänge reduziert werden.

**BEISPIEL 2: Distanzmessung zwischen Genabschnitten von Chromosomen in einem Zellkern mittels mehrdimensionaler Wellenfeldmikroskopie, erfindungsgemäßem Kalibrierverfahren und ggf. Axialtomographie**

(I) In einem Zellkern nimmt das Chromatin der einzelnen Chromsomen definierte Teilregionen ein. Innerhalb einer oder mehrerer solcher chromosomalenTeilregionen werden die zu ortenden Strukturen, d.h. die Meßstrukturen, z.B. kleine Chromosomenabschnitte wie Gene oder Teilstücke von Genen, mit einer im Stand der Technik bekannten Methode der Fluoreszenz in situ Hybridisierung spezifisch markiert, und zwar mit Fluorochromen verschiedener bestimmter spektraler Signaturen $M_1$, $M_2$, $M_3$, .... Die Abstände zwischen den Markierungsorten (den markierten Meßstrukturen) liegen unter der klassischen Auflösung, d.h. sie sind kleiner als die Halbwertsbreite des Hauptmaximum der effektiven Punktbildfunktion. Die Markierung der (Objekt-) Strukturen (Meßstrukturen) erfolgt derart, daß die spektralen Signaturen an den zu ortenden Strukturen (Meßstrukturen) mit nahezu der gleichen Dynamik vertreten sind.

Das biologische Objekt wird auf einer Glasfaser exakt definierten Durchmessers oder in einer runden oder rechteckigen Kapillare definierter Dimensionen präpariert.

(II) Um die Distanzen zu bestimmen, werden mikroskopierbare Präparate mit Kalibriertargets hergestellt, und zwar unter den gleichen physikalischen und chemischen Versuchsbedingungen wie das Objekt bzw. die zu ortenden Objektstrukturen (= Meßstrukturen).

Als Kalibriertargets bzw. als Präparate mit Kalibriertargets dienen beispielsweise:

a) mikroinjizierbare Kügelchen einer spektralen Signatur (monochromatisch):

Die Kügelchen sind nach bekannten Verfahren mit jeweils einem Fluorochrom, d.h. monochromatisch markiert und aufgrund ihrer Größe von den auszumessenden (zu ortenden) Strukturen im Objekt (den Meßstrukturen) unterscheidbar. Man injiziert solche Kalibrierkügelchen, die die im Objekt vorhandenen spektralen Signaturen der Meßstrukturen repräsentieren, ansonsten aber vorzugsweise identisch sind (hinsichtlich Größe, Geometrie, Materialbeschaffenheit etc.). Mit anderen Worten: Die spektralen Signaturen der Meßstrukturen sowie der Kalibriertargets werden so gewählt, daß unter den gegebenen Untersuchungsbedingungen die von ihnen emittierten Fluoreszenzemissionen getrennt voneinander analysiert werden können. Die Injizierung und Fixierung der monochromatischen Kalibrierkügelchen erfolgt derart, daß sich die einzelnen Kügelchen verschiedener spektraler Signatur in Clustern direkt an der (Glasfaser-Oberfläche oder Kapillarwand anordnen, vorzugsweise in einer Querschnittsebene der Faser bzw. Kapillare. Bei Verwendung von Präzisionsfasern und/oder -kapillaren liegen die Kügelchen folglich in definierten Abstände voneinander bzw. von einer Bezugsebene, Bezugsachse oder Bezugslinie.

b) mikroinjizierbare Testkügelchen multispektraler Signatur (polychromatisch) und gleicher spektraler Dynamik:

Die Kügelchen sind nach bekannten Verfahren mit jeweils allen bei den markierten (Objekt-)Strukturen (Meßstrukturen) vorkommenden spektralen Signaturen markiert. Infolgedessen können sie an beliebige Stellen im zu vermessenden biologischen Objekt (hier Kern) injiziert werden. Eine Sollgeometrie wie bei a) ist nicht erforderlich, da für jede Signatur die chromatischen Schwerpunkte an derselben Stelle lokalisiert sein sollen. Zur Unterscheidung von den markierten (Objekt-)Strukturen (Meßstrukturen) können die Kügelchen entweder einer anderen Größenklasse angehören oder aber eine zusätzliche spektrale Signatur tragen, die bei den zu messenden Strukturen d.h. den Meßstrukturen (gemäß Präparationsprotokoll) nicht vorkommt.

c) simultan markierte Chromosomenregionen bekannter Distanz an einem anderen Chromosom als demjenigen, das die zu ortenden Strukturen (Meßstrukturen) trägt:

Die Kalibriertargets, d.h. hier die Chromosomenregionen mit bekanntem Abstand voneinander, sind mit Hilfe einer Probenkombination von DNA-Sequenzen, die die verschiedenen spektralen Signaturen trägt, unterschiedlich markiert. Eine Unterscheidung der chromosomalen Kalibriertargets von den zu ortenden (Chromosomen-) Strukturen (Meßstrukturen) kann beispielsweise durch unterschiedliche Fluoreszenzintensität erfolgen oder durch ein unterschiedliches Intensitätsverhältnis zwischen Fluorochromen verschiedener spektraler Signatur oder durch Verwendung eines zusätzlichen Fluorochroms mit abweichender spektraler Signatur, das bei der Fluoreszenzmarkierung der Meßtargets nicht verwendet wurde. Es ist auch möglich, daß die Kalibriertargets einer anderen (Größenklasse angehören als die zur ortenden Meßstrukturen.

(III) Die Distanzmessungen werden mit einem erfindungsgemäßen mehrdimensionalen Wellenfeldmikroskop, kombiniert mit Photomultiplier und/oder Kamera und Datenverarbeitungsanlage, durchgeführt. Von dem biologische Mikroobjekt, hier im Beispiel einem Zellkern, wird eine Serie optischer Schnitte aufgenommen. Die Meßstrukturen $M_1$, $M_2$, $M_3$,...$M_l$ besitzen $l$ = 1,2,..,$L$ spektrale Signaturen. Die spektrale Signatur der Kalibriertargets $U_1$, $U_2$, $U_3$,. . . ,$U_1$ unterscheiden sich von derjenigen der Meßstrukturen z.B. in Volumen, Durchmesser, Intensität oder in der Zahl der spektralen Signaturen ($l$= 1,2,..,$L$ +1). Die Bilder der optischen Schnitte werden für jede spektrale Signatur getrennt aufgenommen und gegebenenfalls noch der Untergrund korrigiert. Zur Auswertung werden zum einen die Kalibriertargets identifiziert und der chromatische Versatz bestimmt. Dazu werden die Kalibriertargets unter jeder spektralen Signatur lokalisiert und die Abstände zwischen den Kalibriertargets mit Fluorochrommarkierungen verschiedener spektraler Signatur gemessen. Die gemessenen Lokalisationen ( d.h. die gemessenen Targetabstände) werden mit den aufgrund der Geometrie berechneten Soll-Lokalisationen ( d.h. den tatsächlichen Targetabständen) verglichen und daraus der spektral bedingte Versatz (Shift) bestimmt. Dieser Versatz (Shift) ist der Kalibrierwert für die gemessenen Distanzwerte zwischen den zu ortenden (Objekt-)Strukturen (Meßstrukturen).

Da dieser Versatz von den optischen Eigenschaften des Präparates abhängt (z.B. Brechzahlen in den Kernen und dem Präparationsmedium), sollte die Kalibrierung in situ erfolgen. Das heißt im vorliegenden Beispiel, daß sich die Kalibriertargets neben den zu untersuchenden und markierten (chromosomalen) Strukturen (Meßstrukturen) im Kern befinden sollten.

Zum anderen werden die Abstände zwischen den zu ortenden (Objekt-)Strukturen (Meßstrukturen) lokalisiert. Man bestimmt dabei in jeder spektralen Signatur zunächst unabhängig voneinander die Position der Schwerpunkte der gemessenen Intensitätssignale, d.h. es werden die Abstände zwischen den verschiedenen Farbsignalen bzw. Farbpunkten der betreffenden Meßstrukturen, z.B. zwischen dem rotfluoreszierenden und dem grünfluoreszierenden Farbpunkt (von Intensitätsmaximum zu Intensitätsmaximum oder von Schwerpunkt/Baryzentrum zu Schwerpunkt/Baryzentrum) gemessen, und dieser Meßwert wird um den mit den Kalibriertargets ermittelten (durch die verschiedene spektrale Signatur bedingten) Versatz in hochpräziser Weise korrigiert.

Die korrigierten Positionen der Meßstrukturen werden in Bezug auf einen Vergleichspunkt angegeben. Dieser Vergleichspunkt kann z.B. ein beliebig ausgezeichneter fester Punkt im Objekt oder der Schwerpunkt eines Kalibriertargets (z.B. eine markierte Chromosomenregionen) oder eines sonstwie ausgezeichneten Chromosomenterritoriums sein. Es kann aber auch die Schwerpunktskoordinate aller Meßstrukturen innerhalb eines Chromosomenterritoriums sein.

Bei Kalibriertargets in Gestalt von mikroinjizierbaren Testkügelchen mit multispektraler Signatur (polychromatisch) wird der chromatische Versatz aus dem Lokalisationsunterschied der Schwerpunkte für jede Signatur bestimmt. Die dafür erforderliche Identifizierung der zu einem Kalibriertarget gehörenden Fluoreszenzemission kann beispielsweise durch volumenerhaltende Schwellwertverfahren oder durch Mittelung der Segmentierungsergebnisse bei Schwellwertvariation erfolgen.

Bei Kalibriertargets in Gestalt von fluorochrommarkierten Objektregionen mit multispektraler Signatur (polychromatisch) wird der chromatische Versatz genauso bestimmt.

Als fluorochrommarkierte Kalibrierregionen eignen sich insbesondere auch Zentromerregionen, die mit einer Probenkombination von solchen DNA-Sequenzen hybridisiert werden, die alle an dieselben chromosomalen DNA-Abschnitte binden, jedoch mit Fluorochromen unterschiedlicher spektraler Signatur markiert wird. Erfolgt die Hybridisierung unter hoch stringenten Bedingungen, liegen pro Zellkern zwei Markierungsregionen vor; bei nieder-stringenten Bedingungen werden aufgrund zusätzlicher Nebenbindungsregionen zusätzliche Zentromerregionen markiert, so daß die Zahl der Kalibrierungsregionen ansteigt. Das ist u.U. sehr von Vorteil.

(IV) Die beschriebenen Meßverfahren können auch in Kombination mit Axialtomographie durchgeführt werden. Hierfür wird das biologische Mikroobjekt, z.B. ein Zellkern, in dem die zu ortenden Meßstrukturen bereits mit Fluorochromen markiert sind und das auch bereits Kalibriertargets enthält (zur Präparation siehe Beispiel 1), auf einer Glasfaser oder in der Mikrokapillare angeordnet. Mit dem Axialtomograph wird das Objekt Schritt für Schritt um einen definierten Winkel gedreht unter ggf. automatischer Refokussierung. Von jedem Winkelschritt wird ein kompletter 2D oder 3D-Bildstapel aufgenommen.

Die Drehung erfolgt in der Art, daß jeweils ein Abstand zwischen zwei Meßstrukturen bzw. Kalibriertargets (d.h. zwischen deren Fluoreszenzintensitätsschwerpunkten) maximal wird. Der maximale gemessene Abstand entspricht dem tatsächlichen Abstand.

Ist man nur an den Abständen zwischen den Meßstrukturen bzw. Kalibriertargets, d.h. nicht an ihrer absoluten räumlichen Anordnung interessiert, kann man nun von einer der bekannten Meßstrukturen bzw. Kalibriertargets aus fortfahren, den Abstand zu einer dritten Meßstruktur bzw. einem dritten Kalibriertarget zu maximieren und zu bestimmen. Sind die Abstände zwischen den Meßstrukturen bzw. Kalibriertargets größer als die Halbwertsbreite des Hauptmaximums der Punktbildfunktion, so genügt eine einzige spektrale Signatur; sind die Abstände dagegen kleiner, müssen die Meßstrukturen bzw. Kalibriertargets durch multispektrale Signatur unterschieden werden. Die Schwerpunkte (Maxima) der Signale dienen der Lokalisierung. Sofern die untersuchten Meßstrukturen einen Durchmesser haben, der kleiner als die Halbwertsbreite des Hauptmaximums der effektiven Punktbildfunktion ist, werden alle Beugungsbilder der Meßstrukturen bzw. Kalibriertargets durch eine scharfe Punktbildfunktion bestimmt, so daß die Maxima optimal bestimmt werden können.

Ist man an der absoluten Anordnung der Meßstrukturen bzw. Kalibriertargets im Raum interessiert, so müssen die jeweiligen Schwerpunkte (sog. "Baryzentren") präzise bestimmt werden. Durch mehrmaliges Wiederholen der gesamten Meßprozedur und statistische Auswertung kann die absolute Lokalisierung der Meßstrukturen bzw. Kalibriertargets, d.h. die Winkelmessung, verbessert werden.

Anstatt wie vorstehend beschrieben die Kalibrierung und die Distanzmessung zwischen den zu ortenden Strukturen, d.h. Meßstrukturen, in demselben biologischen Objekt durchzuführen, kann man auch die Kalibrierung unabhängig von den Meßstrukturen an gleichartigen biologischen Objekten durchführen. Bei dieser Verfahrensvariante ist die Unterscheidung zwischen den Fluoreszenzsignalen der Kalibriertargets und denjenigen der Meßstrukturen erleichtert. Anhand der mit den Kalibriertargets ermittelten Werte des optischen Versatzes kann man Eichkurven für die Distanzmessungen zwischen den Meßstrukturen erstellen. Derartige Eichkurven machen z.B. Angaben über den spektralen Versatz als Funktion von Brechungsindex und Absorption des verwendeten Immersionsmediums, der verwendeten Optik, Filter und Detektionseinheiten, der verwendeten Auswertealgorithmen, der verwendeten biologischen Objekte, der Lokalisation der Meßstrukturen bzw. Kalibriertargets in ihnen etc. Die Verwendung der Information aus diesen speziellen Eichkurven zur erfindungsgemäßen Distanzmessung bietet sich insbesondere in solchen Fällen an, bei denen einer größere Präzisionstoleranz verwendet erlaubt ist.

**BEISPIEL 3: Untersuchung und Darstellung von dreidimensional ausgedehnten Objekten mittels mehrdimensionaler Wellenfeldmikroskopie, erfindungsgemäßem Kalibrierverfahren und simultaner Bildaufnahme**

Üblicherweise wird von einem biologischen Mikroobjekt ein 3D-Datensatz durch sequentielle Registrierung gewonnen, z.B. in der konfokalen Laserscanningmikroskopie durch punktweises oder linienweises Abscannen des 3D-Objektvolumens; ein zweites Verfahren beruht auf der Registrierung der Fluoreszenzemission aus der Objektebene durch Positionierung eines Detektorarrays in der zu der Objektebene konjugierten Zwischenbildebene. üblicherweise ist die Position dieser Zwischenbildebene fixiert; um 3D-Informationen über das biologische Objekt zu erhalten, wird dieses sequentiell durch die zu der festen Zwischenbildebene konjugierte Objektebene hindurch bewegt; jedesmal wird ein 2D-Bilddatensatz der betreffenden Fluoreszenzemission registriert; und/oder das Objekt wird mit Hilfe axialtomographischer Verfahren um verschiedene Drehwinkel gedreht, wobei jedesmal 2D-Datensätze der zu der festen Zwischenbildebene konjugierten Objektebene registriert werden.

Diese sequentielle Registrierung von Objektpunkten, Objektlinien oder Objektebenen hat verschiedene Nachteile: Z.B. kann ein Ausbleichen der Registrierung des 3D-Datensatzes zu einer Verschiebung der erfindungsgemäß bestimmten 3D-Schwerpunkte der Fluoreszenzverteilung von markierten Objektpunkten einer bestimmten spektralen Signatur führen. Ein anderer Nachteil besteht darin, daß die 3D-Datenaufnahme nicht schnell genug erfolgt, um auch bei nicht permanent stabil, d.h. sich bewegenden Objekten, insbesondere bei in vivo Markierungen wie z.B. bei fluoreszenzmarkierten Chromosomenregionen in Kernen lebender Zellen, die sich unter physiologischen Bedingungen mit Geschwindigkeiten bis zu einigen nm/sec (mittlere Verschiebung) bewegen können, eine befriedigende Bildqualität zu gewährleisten.

Um auch bei sich bewegenden Objekten eine befriedigende Abbildungsqualität zu erhalten, sieht die vorliegende Erfindung eine simultane Aufnahme des 3D-Datensatzes eines fluoreszenzmarkierten Objektes vor. Hierzu wird das vom Objekt emittierte Fluoreszenzlicht einer gegebenen spektralen Signatur durch optische Elemente, z.B. Teilerspiegel, aufgespalten in N Teilstrahlen und auf N Detektorarrays abgebildet, die sich in N verschiedenen Zwischenbildebenen befinden, die zu N verschiedenen Objektebenen konjugiert sind. Eine einfache Abschätzung aufgrund der Abbildungsgleichung ergibt, daß die Abstände der Zwischenbildebenen (Detektorebenen) bei simultaner Registrierung eines Objektbereiches mit 10 $\mu$m axialer Ausdehnung und eines Objektivs von konventioneller Bildweite und hoher numerischer Apertur z.B. um einen Bereich in der Größenordnung $\leq$ 20 cm variiert werden müssen (abhängig vom verwendeten Objektiv). Durch weitere N Zwischenoptiken können die für Hochpräzisionsdistanzmessungen in relevanten Objektbereichen zu registrierenden N Objektebenen auch auf verschiedene Bereiche desselben, geeignet groß dimensionierten Detektorarrays (bzw. auf L < N Detektorarrays) abgebildet werden. In diesem Falle entspricht einem bestimmten Ausschnitt des/der L Detektorarrays eine der N konjugierten Objektebenen. Beispielsweise wird ein kleiner Objektbereich von wenigen $\mu$m Ausdehnung zur Vermessung in der Wellenfeldmikroskopie zunächst so grob positioniert, daß sein Schwerpunkt etwa im Zentrum des durch die Detektionspunktbildfunktion für eine bestimmte (Zwischen)Bildebene $B_0$ gegebenen Beobachtungsvolumens des zur Registrierung verwendeten Mikroskopobjektivs liegt; das vom 3D-Objekt ausgehende Fluoreszenzlicht wird nach seinen spektralen Signaturen getrennt und in N Teilstrahlen aufgespalten, die auf N Detektorarrays (von z.B. je 8 x 8, 16 x 16 oder 64 x 64 Pixelgröße) abgebildet werden, deren Positionierung die Registrierung der Fluoreszenzemission aus N konjugierten Objektebenen um das Maximum der Detektionspunktbildfunktion (bezogen auf die Bild-

ebene $B_0$ ) gestattet.

Beispielsweise sind bei simultaner Aufnahme von Objekten mit einem Abstand von jeweils 20 nm nach einer einfachen Abschätzung unter obigen Annahmen Verschiebungen der konjugierten Zwischenbildebenen um jeweils wenige 100 μm (in der Nähe des Maximums der Punktbildfunktion) erforderlich; bzw. entsprechend kleine optische individuelle Korrekturen sind bei simultaner Detektion der relevanten Objektausschnitte auf einem einzigen (oder L < N) Detektorarray(s) entsprechender Pixelzahlen auszuführen. Bei einer Teilung der Fluoreszenzemission z.B. in N=20 Teilstrahlen gleicher Intensität vermindert sich die Anzahl der von jedem der N=20 Detektorarray(s) (bzw. - Ausschnitte) pro Zeiteinheit registrierten Photonenzahl um etwa den gleichen Faktor. Die Lokalisationsgenauigkeit eines fluoreszenzmarkierten Objektes vermindert sich dann aufgrund der verschlechterten Photonenstatistik um schätzungsweise einen Faktor √20 Dieser Nachteil kann durch eine Erhöhung der Registrierungszeit um den Faktor N (z.B. N=20) beseitigt werden. In diesem Falle dauert die simultane Registrierung des Objektes etwa ebensolange wie bei sequentieller Registrierung. Bei Objekten mit Ausbleichverhalten besteht der Vorteil der simultanen dreidimensionalen Registrierung jedoch in einer für alle Targets (einer gegebenen spektralen Signatur) des Beobachtungsvolumens ähnlichen (bzw. ähnlicherem) Ausbleichverhalten; durch Ausbleichen verursachte Verschiebungen des Schwerpunktes des Fluoreszenzemissionsbildes werden hierdurch vermindert. Bei Objekten mit zeitlich dynamischer Struktur (z.B. markierte Zellen in vivo) wird bei einer gegenüber der sequentiellen Registrierung um den Faktor N verkürzten Aufnahmezeit (z.B. 1 sec statt 20 sec) die Lokalisationsgenauigkeit der einzelnen Objektpunkte um schätzungsweise den Faktor √N (bei N=20 z.B. 4.5) vermindert.

Beispielsweise ergibt sich bei einer 3D Lokalisationsgenauigkeit im Wellenfeldmikroskop von ca. ± 3 nm, erhalten mit sequentiellen Aufnahmezeiten von 1 sec pro Bildebene, unter den genannten Bedingungen bei simultaner Registrierung (1 sec) eine Verminderung auf eine Lokalisationsgenauigkeit auf schätzungsweise ± 4.5 · 3 nm ≈ 14 nm bei simultaner Aufnahme von 20 Objektebenen unter sonst gleichen Bedingungen. Bei einer (als Beispiel) angenommenen Objektbewegung (mittlere Verschiebung) von 5 nm/sec wäre die tatsächliche Lokalisationsungenauigkeit bei einer sequentiellen Aufnahme mit einer Gesamtregistrierungszeit von 20 sec bereits ohne Berücksichtigung von Ausbleicheffekten jedoch erheblich größer. Erfindungsgemäß ist hier die beschriebene simultane Registrierung von Objektebenen nicht nur hinsichtlich einer optischen Achse, sondern auch hinsichtlich zwei und drei orthogonalen optischen Achsen.

Bei Bedarf kann diese erfindungsgemäße simultane Bildaufnahme ohne weiteres mit einer herkömmlichen sequentiellen Bildaufnahme kombiniert werden.

**BEISPIEL 4: DNA-Sequenzierung mittels mehrdimensionaler Wellenfeldmikroskopie**

Mittels bekannter Verfahren wie z.B. der Polymerase-Kettenreaktion werden alle komplementären Subsequenzen der zu analysierenden DNA-Sequenz herstellt. Die Subsequenzen beginnen alle am selben Nukleotid der zu analysierenden Sequenz. Die zu analysierenden Bruchstücke werden alle am 3' Ende mit einem Bezugsfluorochrommarker α markiert. Am anderen Ende, dem 5'-Ende, bzw. an definierten Zwischenstellen werden sie jeweils mit einem Fluorochrommarker a, g, c, t verschiedener spektraler Signatur markiert, je nachdem ob das Nukleotid die Base Adenin (Marker a), Guanin (Marker g), Cytosin (Marker c) oder Thymin (Marker t) trägt.

Alle Typen der verwendeten Fluorochrommarker unterscheiden sich in ihrer spektralen Signatur derart, daß die Fluorochrommarker α, a, g, c, t (ggf. noch weitere) getrennt voneinander detektiert werden können; ein bestimmter Fluorochrommarker kann erfindungsgemäß von einem bis vielen Fluorochrommolekülen des gleichen oder verschiedenen Typs gebildet werden, wobei die Länge und Zusammensetzung der Fluorochrommarker erfindungsgemäß so gewählt wird, daß Distanzmessungen zwischen den Schwerpunkten der Intensitätsverteilungen von anfangsständigem Fluorochrommarker α und endständigen Fluoreszenzmarkern (entweder a, oder g, oder c, oder t, oder ggf. weitere für weitere Basen) mit dem Verfahren der mehrdimensionalen Wellenfeldmikroskopie möglich ist, d.h.. Linkermoleküle für Fluoreszenzmarker müssen dabei beispielsweise kürzer als 1/2 Nukleotiddurchmesser sein. Erfindungsgemäß ist auch die Verwendung von längeren Linkermolekülen möglich, sofern diese eine Konfiguration von so hoher Steifigkeit besitzen, daß die durch sie verursachte Distanzvariation genügend klein ist, beispielsweise < 1/2 Nukleotiddurchmesser beträgt. Diese so markierten Subsequenzen repräsentieren die zu analysierende DNA-Sequenz vollständig. Die Fluoreszenzmarker a, g, c, t bzw. das Bezugsfluorochrom α können jeweils ein oder mehrere Fluorochrommoleküle enthalten. Die DNA-Subsequenzen werden alle so auf geeignete Träger fixiert, daß sie als lineare Sequenz vorliegen.

Die fluorochrommarkierten DNA-Stücke werden mit Hilfe von DNA-Combing-Techniken linear ausgerichtet. Im Unterschied zum herkömmlichen "eindimensionalen" Wellenfeldmikroskop ist bei dem erfindungsgemäßen mehrdimensionalen Wellenfeldmikroskop eine zusätzliche präzise Ausrichtung der DNA-Stränge in Richtung der optischen Achse des Systems nicht mehr notwendig. Die DNA-Sequenzen werden vorzugsweise auf eine viereckige Glasfaser aufgebracht, deren Brechungsindex sich von demjenigen des umgebenden Mediums in minimaler Weise unterscheidet, wobei die Ausrichtung in einem bestimmten Winkel, insbesondere orthogonal zur Achse der Glasfaser erfolgt, und wobei der mittlere Abstand der Schwerpunkte der DNA-Sequenzen voneinander größer ist als die maximale Halbwertsbreite der zur Registrierung der Fluoreszenzsignale verwendeten Punktbildfunktionen. Ein weiteres Verfahren bindet das 3' Ende an ein Mikrokügelchen der spektralen Signatur α und streckt anschließend den DNA-Faden mit dem Werkzeug der optischen Pinzette, wobei der "Pinzettenlaser" in seiner Wellenlänge geeignet gewählt werden muß.

Nach Linearisierung bzw. Orientierung der DNA-Sequenzen wird das so hergestellte Präparat fixiert und die molekulare Bewegung z.B. durch Temperaturerniedrigung reduziert. Alternativ können die DNA-Enden auch in eine kristallin geordnete Festkörperstruktur eingebettet werden. Für die Messung wurden zu Zwecken der Kalibrierung weitere, insbesondere polychromatische Mikroobjekte auf die Glasfaser, den DNA-Objektträger oder in den Fixierungsfestkörper der DNA-Enden eingebracht. Die Kalibrierungsobjekte enthalten zusätzlich eine spektrale Signatur, die nicht a, t, c oder g ist. Die Linearisierung der DNA-Stränge kann entfallen, wenn alle Nukleotiden bei der Synthese des zu analysierenden DNA-Komplementärstranges geeignet fluoreszenzmarkiert werden.

Die fixierten DNA-Sequenzen werden in ein mehrdimensionales Wellenfeldmikroskop eingebracht, wobei die linearen DNA-Subsequenzen so zu den stehenden Wellenfronten orientiert werden, daß eine exakte Abstandsmessung (Genauigkeit $\leq 1 \cdot 10^{-10}$ m) zwischen α und a bzw. g, c oder t — nach Bestimmung der Intensitätsbaryzentren und Kalibrierung der Abbildungseigenschaften — möglich ist.

Die Messung erfolgt bei in situ Kalibrierung unter Anwendung des erfindungsgemäßen Kalibrierverfahrens. Die Signale der Fluorochrommarker werden im Wellenfeldmikroskop mit geeignet angepaßten Schrittweiten spektral getrennt voneinander registriert (bevorzugt in digitaler Weise). Aus den Abständen der fluoreszenten Markern und ihrer spektralen Signatur läßt sich die DNA-Basensequenz des zu analysierenden DNA-Stückes bestimmen.

Statt einer spektralen Trennung oder zusätzlich zu ihr können auch Fluoreszenzlebensdauerparameter analysiert werden. In einer ersten Phase der Auswertung wird eine Grobbestimmung der Schwerpunkte der Fluorochrommarker-Signale vorgenommen und auf der Grundlage dieser Information die zu einer DNA-Sequenz gehörenden Signale nach den oben genannten Distanzkriterien von den zu einer anderen DNA-Sequenz gehörenden Signalen getrennt; in einer zweiten Phase der Auswertung werden die spektral getrennt registrierten, modulierten Signale der Fluorochrommarker mit Hilfe von geeignet angepaßten Funktionen analysiert; hieraus werden die Abstände z.B. der Schwerpunkte der anfangs- und endständigen Fluorochrommarker-Signale einer DNA-Sequenz voneinander mit molekularer Präzision bestimmt, wobei die an den Kalibrierungsobjekten vorgenommenen Messungen für eine Korrektur von Distanzaberrationen, z.B. chromatischer Verschiebungen, herangezogen werden; in einer dritten Phase der Auswertung werden die den Längen der DNA-Sequenzen entsprechenden Abstände der Fluorochrommarker-Signale nach zunehmender Länge getrennt nach Typus des endständigen Fluorochrommarkers (z.B. a, g, c, t) geordnet; die so erreichte Anordnung entspricht dem bei einem herkömmlichen Verfahren erzielten Muster, dem dann nach bekannten Methoden die gewünschte Sequenzinformation entnommen werden kann.

Bei Makromolekülen linearer Sequenz bzw. bekannter geordneter Struktur geht man analog vor, wobei die Zahl und Art der Fluorochrommarker von Zahl und Art der Molekülbausteine abhängt.

Falls die Vermessung einzelner DNA-Stränge deren Ausrichtung in Richtung der optischen Achse erforderlich macht, kann erfindungsgemäß wie folgt verfahren werden: Das bekannte Ende der DNA-Kette wird zusätzlich zu einem Fluoreszenzmarker der spektralen Signatur α mit einem chemischen "Marker" markiert. Der Rest der DNA-Kettenpräparation, insbesondere die Markierung der endständigen Fluoreszenzmarker a, c, g und t (Stopnukleotide) wird wie anfangs beschrieben durchgeführt. Die endständigen Basen und/oder die Marker der Stopnukleotide und ggf. weitere Basen der auszurichtenden DNA-Ketten tragen eine elektrische Ladung (z.B. negativ).

Die Ausrichtung der DNA-Ketten kann vor oder während der Mikroskopie erfolgen, zunächst wird das Verfahren der Ausrichtung vor der Mikroskopie beschrieben.

Die präparierten DNA-Ketten werden in Lösung in einem Puffer niedriger Ionenstärke auf einen speziell beschichteten Objektträger (oder Deckglas, was im folgenden auch als Objektträger bezeichnet werden soll) aufgebracht, der das chemisch markierte 3' Ende jeder DNA-Kette binden ("ankleben") kann. Der Lösung wird nun eine immobilisierende Komponente zugegeben, die nach einer bestimmten Zeit eine Aushärtung der DNA-Ketten in Lösung bewirkt. Der Objektträger wird mit einem (unbeschichteten) Deckglas abgedeckt und versiegelt, wobei der Abstand von Objektträger zum Deckglas mit einem geeigneten "Spacer" (z.B. einer dünnen Membran) auf einen wohldefinierten Wert eingestellt werden kann. Der so versiegelte Objektträger wird einem geeigneten homogenen, statischen elektrischen Feld ausgesetzt, welches die elektrisch geladenen Basen ausrichtet. Die Polung des elektrischen Feldes (beispielsweise des eines Kondensators) muß hierbei so angelegt sein, daß bei negativ (positiv) geladenen Basen die Kathode (Anode) bei dem beschichteten Objektträger (mit den "angeklebten" 3' Enden) und die Anode (Kathode) bei dem unbeschichteten Deckglas liegt. Die elektrischen Feldlinien verlaufen senkrecht zur Objektträgeroberfläche und die Stärke des elektrischen Feldes wird ausreichend hoch gewählt, um eine Ausrichtung der DNA-Ketten in der Lösung zu bewirken.

Nachdem die so ausgerichteten DNA-Ketten in dem Zwischenraum Objektträger - Deckglas immobilisiert sind, werden sie mit dem mehrdimensionalen Wellenfeldmikroskop wie vorstehend beschrieben aufgenommen bzw. vermessen.

Soll die Orientierung der DNA-Ketten während der mikroskopischen Aufnahme erfolgen, wird das oben beschriebenen elektrische Feld erfindungsgemäß nach dem folgenden Verfahren aufgebaut:

Der beschichtete Objektträger ist ein elektrisch leitender Spiegel hinreichender Planität. Die Lösung der DNA-Ketten (niedrige Ionenstärke) ist geeignet viskos und es werden keine immobilisierenden Komponenten der Lösung zugegeben. Auch hier wird wieder (ggf. unter Verwendung eines geeigneten Spacers) ein Deckglas aufgebracht und versiegelt. Das Wellenfeld wird nun mit nur einem Objektiv in Verbindung mit dem Spiegel erzeugt, wobei das aus dem Objektiv austretende Anregungslicht vom Spiegel reflektiert wird und sich ein eindimensionales Wellenfeld (parallel zur Fokalebene oder zur Spiegeloberfläche) ausbildet. Das Anlegen einer positiven (negativen) Spannung bei der Verwendung von negativ (positiv) geladenen Basen bewirkt ebenfalls eine Ausrichtung der DNA-Ketten senkrecht zur Oberfläche des Spiegels, also entlang der optischen Achse des Mikroskops und die nun wie vorstehend beschrieben sequenziert werden können. Auch bei diesem Verfahren muß die Stärke des elektrischen Feldes groß genug sein, um die Ausrichtung der DNA-Ketten zu bewirken; thermische Bewegungen der Moleküle können z.B. durch Temperaturerniedrigung reduziert werden.

Analog diesem Beispiel kann mit beliebigen anderen linearisierten Makromolekülen verfahren werden.

**BEISPIEL 5: Mehrdimensionales Wellenfeldmikroskop Typ II mit lateraler räumlich modulierter Fluoreszenzanregung**

Das mehrdimensionale Wellenfeldmikroskop Typ II umfaßt in der Raumrichtungen x eine reelle Beleuchtungsquelle für kohärenzfähige Lichtstrahlen, einen Strahlteiler zur Auskopplung von Teilstrahlen als virtuelle Beleuchtungsquelle, und ein erstes Objektiv, das diesen beiden Beleuchtungsquellen zugeordnet ist. In dieses erste Objektiv werden die Lichtstrahlen bzw. Lichtwellenzüge der Beleuchtungsquellen derart eingekoppelt, daß sie auf der hinteren, dem Objektraum abgewandten Fokalebene (diese Ebene wird auch "back focal plane" genannt.) zwei voneinander beabstandete Fokuspunkte erzeugen bzw. aufweisen und in dem Raum zwischen den beiden Fokalebenen in einem bestimmten Winkel aufeinander zu verlaufen und zu einem eindimensionalen stehenden Wellenfeld interferieren:

Fokussiert man einen Lichtstrahl (Lichtwellenzug) mit einem geeigneten Abstand zur optischen Achse in diese Fokalebene, (die optische Achse steht senkrecht auf der Fokalebene und verläuft durch deren Mittelpunkt) so verläßt im Objektraum ein paralleles Lichtbündel mit ebenen Wellenfronten das Objektiv, und zwar unter einem bestimmten Winkel zur optischen Achse. Dieser Winkel ist variabel einstellbar, je nach dem, mit welchem Abstand zu optischer Achse die Lichtstrahlen in das Objektiv eingekoppelt werden und um welche Art von Objektiv es sich handelt.

Koppelt man den zweiten Lichtstrahlen (Lichtwellenzüge) unter einem solchen Winkel zu dem ersten und zur optischen Achse in dasselbe Objektiv ein, daß sein Fokuspunkt auf der hinteren Fokalebene diametral gegenüber dem Fokuspunkt des ersten Lichtstrahls liegt, daß also Fokuspunkt 1 — optische Achse — Fokuspunkt 2 eine Linie auf der hinteren Fokalebene bilden, entsteht in dem Raum zwischen den beiden Fokalebenen ein zweites paralleles Lichtbündel mit ebenen Wellenfronten, das in einem bestimmten Winkel zu dem ersten verläuft und mit diesem im Objektraum zu einem eindimensionalen stehenden Wellenfeld mit Streifen maximaler Lichtintensität interferiert.

Dem ersten Objektiv ist ein zweites Objektiv mit Abstand spiegelbildlich gegenüber angeordnet, so daß diese beiden Objektive auf zwei gegenüber liegenden Seiten des dreidimensionalen Objektraums liegen. Diesem zweiten Objektiv ist eine dritte und eine vierte (reelle oder virtuelle) Beleuchtungsquelle für kohärenzfähige Lichtstrahlen so zugeordnet, daß man die Lichtstrahlen beider Beleuchtungsquellen — wie für das erste Objektiv beschrieben — auf die hintere, d.h. dem Objektraum abgewandte Fokalebene dieses zweiten Objektives fokussieren kann, in dem Raum zwischen den beiden Fokalebenen dieses zweiten Objektives zu einem eindimensionalen stehenden Wellenfeld interferieren lassen kann, und im Objektraum mit dem eindimensionalen stehenden Wellenfeld des ersten Objektives so zur Interferenz bringen kann, so daß ein dreidimensionales Wellenfeld entsteht, mit Punkten maximaler Intensität, die sich im dreidimensionalen Raum fortsetzen.

Für die Erzeugung eines zweidimensionalen Wellenfeldes (d.h. Punkte maximaler Intensität in einer Ebene), wird der beschriebene Aufbau insoweit abgewandelt, daß man

entweder erstes und zweites Objektiv einsetzt, eines davon aber nur mit einer Beleuchtungsquelle kombiniert,

oder man setzt nur ein einziges Objektiv ein und kombiniert dieses mit einer dritten Beleuchtungsquelle, deren kohärenzfähige Lichtstrahlen man derart zu den Lichtstrahlen der beiden anderen Beleuchtungsquellen in dieses einzige Objektiv einkoppelt, daß die korrespondierenden drei Fokuspunkte auf der Fokalebene ein gleichschenkliges Dreieck bilden, durch dessen Mittelpunkt die optische Achse verläuft. Im Objektraum verlassen alle drei Lichtstrahlen das Mikroskopobjektiv mit dem gleichen Winkel zur optischen Achse aber jeder in eine andere Richtung.

Diese Variante des erfindungsgemäßen Wellenfeldmikroskops Typ II zur Erzeugung eines mehrdimensionalen Wellenfeldmikroskops unter Verwendung eines einzigen Objektivs kann auch zur Erzeugung eines dreidimensionalen Wellenfelds eingesetzt werden. Dazu lenkt man vier Lichtstrahlen in dasselbe Objektiv und zwar derart, daß die dazu korrespondierenden vier Fokuspunkte in der hinteren Fokalebene ein gleichseitiges Viereck bilden.

**Patentansprüche**

1. Wellenfeldmikroskop mit einem Beleuchtungs- bzw. Anregungssystem, das eine Beleuchtungsquelle, ein erstes Objektiv und ein zweites Objektiv oder einen Reflektor umfaßt, wobei erstes Objektiv und zweites Objektiv oder Reflektor derart zueinander angeordnet sind, daß sie zur Erzeugung eines eindimensionalen stehenden Wellenfeldes geeignet sind, mit einem Objektraum, der Halte und Manövriervorrichtung(en) für ein Objekt umfaßt, und mit einem Detektionssystem, das ein Objektiv, ein Okular und einen Detektor umfaßt, dadurch gekennzeichnet,

   daß das Beleuchtungs- bzw. Anregungssystem in zwei oder allen drei Raumrichtungen wenigstens eine reelle oder virtuelle Beleuchtungsquelle für kohärenzfähige Lichtstrahlen und wenigstens einen Reflektor oder Strahlteiler zur Auskopplung von Teilstrahlen oder eine weitere Beleuchtungsquelle für kohärenzfähige Lichtstrahlen umfaßt, denen jeweils wenigstens ein Objektiv zugeordnet ist, und die jeweils zur Erzeugung von Lichtwellenzügen geeignet sind, wobei die Lichtwellenzüge der einen Beleuchtungsquelle antiparallel oder in variabel einstellbaren Winkeln zu den Lichtwellenzügen des Reflektors bzw. der anderen Beleuchtungsquelle ausgerichteten sind, derart, daß die von der einen Beleuchtungsquelle ausgesendeten Lichtwellenzüge mit denen des Reflektors bzw. der anderen Beleuchtungsquelle zu einem stehenden Wellenfeld mit ebenen Wellenfronten interferieren,
   und daß das Detektionssystem wenigstens ein zur epifluoreszenten Detektion geeignetes und/oder wenigstens ein zur rasternden Punktdetektion geeignetes Detektionsobjektiv vorzugsweise hoher numerischer Apertur, welches mit seiner optischen Achse senkrecht zu den Wellenfronten eines der interferierenden Wellenfelder angeordnet ist, umfaßt, das auch mit einem Objektiv des Anregungssystems identisch sein kann, wobei dem zur epifluoreszenten Detektion geeigneten Detektionsobjektiv ein flächiger (zweidimensionaler) Detektor, z.B. eine Kamera vorgeordnet ist, und dem zur rasternden Punktdetektion geeigneten Detektionsobjektiv wenigstens eine feststehende konfokale Detektionsringblende und/oder -lochblende und/oder wenigstens ein feststehender Detektionsschlitz vorgeordnet und ein Punktdetektor, insbesondere ein Photomultiplier, eine Photodiode oder eine Diodenarray nachgeordnet ist.

2. Wellenfeldmikroskop nach Anspruch 1, dadurch gekennzeichnet,

   daß in wenigstens einer Raumrichtung einem Objektiv hoher numerischer Apertur ein Objektiv niedriger numerischer Apertur oder ein Reflektor zugeordnet ist, und in einer oder beiden anderen Raumrichtung(en) entweder zwei Objektive niedriger numerischer Apertur oder ein Objektiv niedriger numerischer Apertur und ein Reflektor einander zugeordnet sind.

3. Wellenfeldmikroskop mit einem Beleuchtungs- bzw. Anregungssystem, das eine Beleuchtungsquelle und ein Objektiv umfaßt, die derart zueinander angeordnet sind, daß sie zur Erzeugung eines stehenden Wellenfeldes geeignet sind, mit einem Objektraum, der Halte- und Manövriervorrichtung(en) für ein Objekt umfaßt, und mit einem Detektionssystem, das ein Objektiv, ein Okular und einen Detektor umfaßt, dadurch gekennzeichnet,

   daß das Beleuchtungs- bzw. Anregungssystem in wenigstens einer der drei Raumrichtungen wenigstens eine reelle oder virtuelle Beleuchtungsquelle für kohärenzfähige Lichtstrahlen und wenigstens einen Strahlteiler zur Auskopplung von wenigstens einem Teilstrahl aufweist, denen ein gemeinsames Objektiv zugeordnet ist, in das die Lichtstrahlen bzw. Lichtwellenzüge der Beleuchtungsquelle(n) und des/der Strahlteiler(s) derart einkoppelbar sind, daß sie auf der hinteren (dem Objektraum abgewandten) Fokalebene zwei voneinander beabstandete Fokuspunkte aufweisen und in dem Raum zwischen den beiden Fokalebenen in variabel einstellbarem Winkel zueinander verlaufen und zu einem eindimensionalen stehenden Wellenfeld interferieren,
   und daß das Detektionssystem wenigstens ein zur epifluoreszenten Detektion geeignetes und/oder wenigstens ein zur rasternden Punktdetektion geeignetes Detektionsobjektiv vorzugsweise hoher numerischer Apertur umfaßt, das auch mit einem Objektiv des Anregungssystems identisch sein kann, wobei dem zur epifluoreszenten Detektion geeigneten Detektionsobjektiv ein flächiger (zweidimensionaler) Detektor, z.B. eine Kamera vorgeordnet ist, und dem zur rasternden Punktdetektion geeigneten Detektionsobjektiv wenigstens eine feststehende konfokale Detektionsringblende und/oder -lochblende und/oder wenigstens ein feststehender Detektionsschlitz vorgeordnet und ein Punktdetektor, insbesondere ein Photomultiplier, eine Photodiode oder eine Diodenarray nachgeordnet ist

4. Wellenfeldmikroskop nach Anspruch 3, dadurch gekennzeichnet,

daß das Beleuchtungs- bzw. Anregungssystem in derselben oder einer oder beiden anderen Raumrichtung(en) jeweils wenigstens eine weitere reelle oder virtuelle Beleuchtungsquelle für kohärenzfähige Lichtstrahlen und/oder wenigstens einen Strahlteiler zur Auskopplung von wenigstens einem Teilstrahl aufweist, dem/denen jeweils ein weiteres Objektiv zugeordnet ist, durch das der/die Lichtstrahlen (Lichtwellenzüge) in den Objektraum gelenkt und derart ausgerichtet sind, daß sie mit den Lichtstrahlen aus derselben oder aus der /den beiden anderen Raumrichtung(en) bzw. dem von diesen gebildeten ein- oder zweidimensionalen Wellenfeld zu einem zwei- bzw. dreidimensionalen Wellenfeld interferieren.

5. Wellenfeldmikroskop nach einem der Ansprüche 1 bis 4, dadurch gekennzeichnet,

daß der Objektraum eine Objekthalterung umfaßt, in bzw. an der das Objekt mit den Meßstrukturen und/oder gegebenenfalls Kalibriertarget(s) um eine oder zwei orthogonal zueinander verlaufende Achsen drehbar in dem Wellenfeld gelagert ist, wobei für wenigstens eine Achse eine Drehbarkeit um 360-Grad ($2\pi$) bevorzugt ist.

6. Wellenfeldmikroskop nach einem der Ansprüche 1 bis 5, dadurch gekennzeichnet,

daß die das mehrdimensionale Wellenfeld erzeugende(n) Beleuchtungsquellen und/oder der/die Reflektoren und/oder der/die Strahlteiler und/oder das/die Objektiv(e) und damit das mehrdimensionale Wellenfeld um eine oder zwei orthogonal zueinander verlaufende Achsen drehbar ist/sind.

7. Wellenfeldmikroskop nach einem der Ansprüche 1 bis 6, dadurch gekennzeichnet,

daß im Detektionssystem ein Scannerspiegel vorgesehen und derart angeordnet ist, daß er zur Abbildung der lateralen Objektbereiche mit der gewünschten, vorzugsweise maximalen Fluoreszenzintensität geeignet ist.

8. Wellenfeldmikroskop nach einem der Ansprüche 1 bis 7, dadurch gekennzeichnet,

daß das Beleuchtungssystem in wenigstens einer der drei Raumrichtungen eine reelle Beleuchtungsquelle für die Zwei- oder Mehrphotonenanregung und in einer oder beiden anderen Raumrichtung(en) eine reelle und/oder virtuelle Beleuchtungsquelle für die Zwei- oder Mehrphotonenanregung umfaßt, und daß die damit erzeugten stehenden Wellenfelder ($WF_1$, $WF_2$, ..., $WF_i$) voneinander verschiedene Wellenlängen ($\lambda_1$, $\lambda_2$ ..., $\lambda_i$) und Distanzen ($d_1$, $d_2$, ..., $d_i$) zwischen den jeweiligen Wellenmaxima bzw. Wellenminima von $d_1 = \lambda_1 / 2n \cos\theta_1$ bzw. $d_2 = \lambda_2 / 2n \cos\theta_2$ bzw. $d_i = \lambda_i / 2n \cos\theta_i$ aufweisen (mit: n = Brechungsindex im Objektraum, $\theta_1$, $\theta_2$,... $\theta_i$ = Kreuzungswinkel des Lichtwellenzugs der Wellenlänge $\lambda_1$, $\lambda_2$ ...,$\lambda_i$ mit der optischen Achse), und wobei die Wellenfelder $WF_1$,$WF_2$ ... $W_i$ derart zueinander ausgerichtet sind, daß sich mindestens ein Maximum zweier oder aller stehenden Wellen an derselben Stelle (nämlich dem Ort einer Mehrphototonenanregung) befindet.

9. Wellenfeldmikroskop nach einem der Ansprüche 1 bis 8, dadurch gekennzeichnet,

daß eine Anordnung aus Beleuchtungsquelle, Objektiv und einem elektrisch leitenden Spiegel, die zur Erzeugung eines eindimensionalen, elektrischen Wellenfeldes geeignet ist, relativ zur Objektträgerhalterung vorgesehen ist, und zwar derart, daß die in dem Objekt befindlichen Meßstrukturen und/oder Kalibriertargets durch Anlegen des elektrischen Feldes — vor oder während des mikroskopischen Meßvorgangs — ausrichtbar sind.

10. Wellenfeldmikroskopieverfahren zur DNA-Sequenzierung unter Verwendung eines Wellenfeldmikroskops nach einem der Ansprüche 1 bis 9, gekennzeichnet durch folgende Verfahrensschritte:

es werden alle komplementären Subsequenzen der zu analysierenden DNA-Sequenz derart hergestellt, daß alle Subsequenzen am selben Nukleotid der zu analysierenden Sequenz beginnen,

die zu analysierenden Bruchstücke werden alle am 3'-Ende mit einem Bezugsfluorochrommarker α und am 5'-Ende und/oder an definierten Zwischenstellen mit einem Fluorochrommarker a, g, c,oder t - je nachdem ob das Nukleotid die Base Adenin (Marker a), Guanin (Marker g), Cytosin (Marker c) oder Thymin (Marker t) trägt — markiert, wobei die Fluorochrommarker a, g, c, t und α verschiedene spektrale Signatur aufweisen, und jeweils ein oder mehrere Fluorochrommoleküle enthalten,

die markierten DNA-Subsequenzen werden derart auf Träger fixiert, daß sie als lineare Sequenz vorliegen, und in ein mehrdimensionales Wellenfeldmikroskop eingebracht, wobei die linearen DNA-Subsequenzen so zu den stehenden Wellenfronten orientiert werden, daß eine exakte Abstandsmessung (Genauigkeit ≤1·10⁻¹⁰ m) zwischen α und a bzw. g, c oder t nach Bestimmung der Intensitätsbaryzentren und Kalibrierung der Abbildungseigenschaften durchführbar ist,

indem die Signale der Fluorochrommarker schrittweise, spektral getrennt voneinander registriert werden,

und aus den Abständen der fluoreszenten Markern und ihrer spektralen Signatur die DNA-Basensequenz des zu analysierenden DNA-Stückes bestimmt wird.

**11.** Kalibrierverfahren für die mehrdimensionale Wellenfeldmikroskopie nach einem der Ansprüche 1 bis 10, bei dem

vor, während oder nach der Präparation des betreffenden Objekts auf einem bzw. in einem Objekthalter, insbesondere Objektträgerplättchen, Objektträgerfaser, Objektträgerkapillare oder Objektträgerflüssigkeit, die zu untersuchenden bzw. zu ortenden Objektstrukturen - ist gleich Meßstrukturen - mit Fluoreszenzfarbstoffen verschiedener oder gleicher spektraler Signatur markiert werden, wobei zumindest solche zu ortenden Meßstrukturen, deren Abstand voneinander geringer ist als die Halbwertsbreite des Hauptmaximums der effektiven Punktbildfunktion, mit Fluoreszenzfarbstoffen verschiedener spektraler Signatur markiert werden,

mit den gleichen Fluoreszenzfarbstoffen Kalibriertargets definierter Größe und räumlicher Anordnung markiert werden,

die fluoreszierenden Kalibriertargets entweder zusammen mit den Objekten bzw. Meßstrukturen oder separat auf bzw. in dem/ einem Objekthalter präpariert werden,

Meßstrukturen und Kalibriertargets unter übereinstimmenden Bedingungen, gleichzeitig oder nacheinander mikroskopisch untersucht werden,

und bei dem jeweils zwei definierte Kalibriertargets verschiedener spektraler Signatur unter Berücksichtigung des wellenlängenabhängigen Abbildungs- und Lokalisationsverhaltens des jeweiligen optischen Systems vermessen werden, die dabei ermittelten Messwerte — gleich Ist-Werte — mit den vorbekannten tatsächlichen Distanzwerten — gleich Soll-Werten — verglichen werden, und aus der Differenz zwischen Ist-Werten und Soll-Werten ein Korrekturwert — ist gleich Kalibrierwert — bestimmt wird, mit dem der durch das optische System bedingte Versatz in der Detektion unterschiedlicher Emissionsloci, insbesondere der Meßstrukturen, korrigiert wird. dadurch gekennzeichnet,

daß das biologische Objekt mit den fluorochrommarkierten Meßstrukturen und/oder fluorochrommarkierten Kalibriertarget(s) sequentiell oder simultan mit einzelnen (separaten), in zwei oder drei Raumrichtungen orthogonal zueinander verlaufenden, stehenden und zu einem zwei- oder dreidimensionalen Wellenfeld miteinander interferierenden Wellenfeldern beleuchtet wird, wobei die Fluorochrome zur Fluoreszenzemission angeregt werden,

daß zur Detektion der Fluoreszenzintensität eine Kamera und/oder eine oder mehrere zweidimensionale Anordnung(en) aus Einzeldetektoren mit jeweils kreis-, ring- oder schlitzförmiger Blende oder eine Anordnung von mehreren kreis-, ring- oder schlitzförmigen Blenden verwendet wird/werden,

daß entweder das Objekt mit den Meßstrukturen und/oder Kalibriertarget(s) oder das ein- bzw. zweidimensionale Wellenfeld oder beides während des Meßvorgangs schrittweise um eine oder zwei orthogonal zueinander verlaufende Achsen gedreht wird, wobei die fluorochrommarkierten Meßstrukturen und/oder Kalibriertargets sequentiell oder simultan mit einem oder zwei einzelnen, orthogonal zueinander stehenden Wellenfeldern beleuchtet werden.

**Europäisches Patentamt**

## EUROPÄISCHER RECHERCHENBERICHT

**Nummer der Anmeldung**

EP 98 12 3687

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| E | WO 99 02974 A (RUPRECHT-KARLS-UNIVERSITÄT HEIDELBERG) 21. Januar 1999<br>* das ganze Dokument *<br>--- | 1-11 | G01N21/64<br>G02B21/06<br>C12Q1/68 |
| A | WO 94 18594 A (CARNEGIE MELLON UNIVERSITY) 18. August 1994<br>* Seite 3, Zeile 12 - Seite 4, Zeile 2 * | 1 | |
| X | * Seite 13, letzter Absatz - Seite 16, Zeile 17 *<br>* Seite 18, Zeile 11 - Seite 22, | 3,7 | |
| Y | Zeile 11 *<br>* Seite 23, Zeile 1 - Seite 25, Zeile 18; Abbildungen 1-8 *<br>--- | 10 | |
| Y | EP 0 732 584 A (IBM) 18. September 1996<br>* Spalte 5, Zeile 28 - Zeile 42 *<br>* Spalte 13, Zeile 39 - Zeile 45 *<br>* Spalte 14, Zeile 7 - Zeile 28 *<br>* Seite 16, Zeile 41 - Spalte 17, Zeile 7 *<br>* Spalte 17, Zeile 17 - Zeile 22 *<br>* Abbildung 8 *<br>--- | 10 | |
| A | WO 96 31522 A (NEW YORK UNIVERSITY) 10. Oktober 1996<br>* Seite 4, letzter Absatz *<br>* Seite 12, Zeile 18 - Zeile 20 *<br>* Seite 57, Zeile 13 - Zeile 15 *<br>* Seite 70, Zeile 18 - Zeile 26 *<br>* Seite 71, Zeile 12 - Seite 72, Zeile 33 *<br>* Seite 92, Absatz 1 *<br>* Seite 140, Zeile 1 - Zeile 3 *<br>--- | 10,11 | **RECHERCHIERTE SACHGEBIETE (Int.Cl.6)**<br><br>G01N |

-/--

Der vorliegende Recherchenbericht wurde für alle Patentansprüche erstellt

| Recherchenort | Abschlußdatum der Recherche | Prüfer |
|---|---|---|
| DEN HAAG | 27. Mai 1999 | Thomas, R.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

**Europäisches Patentamt**

# EUROPÄISCHER RECHERCHENBERICHT

Nummer der Anmeldung

EP 98 12 3687

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | B.BAILEY et al: "Optical subsectioning and multiple focal-plan imaging in the standing-wave fluorescence microscope" PROCEEDINGS 52nd ANNUAL MEETING OF THE MICROSCOPY SOCIETY OF AMERICA, New Orleans, US, 31 July - 5 August 1994, pages 158-159 XP002085004 * Seite 159, Zeile 15 - Zeile 17 * | 11 | |
| A | WO 96 24082 A (UNIVERSITY OF CALIFORNIA) 8. August 1996 * Seite 3, Zeile 14 - Seite 4, Zeile 11 * * Seite 6, Zeile 26 - Seite 7, Zeile 6 * * Seite 15, Zeile 6 - Seite 17, Zeile 27; Abbildungen 2-6 * | 1 | |
| D,A | W. DENK ET AL: "Two-photon laser scanning fluorescence microscopy" SCIENCE., Bd. 248, Nr. 4951, 6. April 1990, Seiten 73-76, XP000381741 Washington DC * Zusammenfassung * | 8 | |
| D,A | US 4 621 911 A (LANNI) 11. November 1986 | | **RECHERCHIERTE SACHGEBIETE (Int.Cl.6)** |
| A | V. KRISHNAMURTHI ET AL: "Image processing in 3-D standing-wave fluorescence microscopy" PROCEEDINGS SPIE - THE INTERNATIONAL SOCIETY FOR OPTICAL ENGINEERING, Bd. 2655, - 1996 Seiten 18-25, XP002085003 | | |

Der vorliegende Recherchenbericht wurde für alle Patentansprüche erstellt

| Recherchenort | Abschlußdatum der Recherche | Prüfer |
|---|---|---|
| DEN HAAG | 27. Mai 1999 | Thomas, R.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 98 12 3687

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-05-1999

| Im Recherchenbericht angeführtes Patentdokument | Datum der Veröffentlichung | Mitglied(er) der Patentfamilie | Datum der Veröffentlichung |
|---|---|---|---|
| WO 9902974 A | 21-01-1999 | AU 9251898 A<br>DE 19830596 A | 08-02-1999<br>14-01-1999 |
| WO 9418594 A | 18-08-1994 | US 5394268 A<br>AU 691173 B<br>AU 6169994 A<br>CA 2155521 A<br>EP 0682780 A<br>JP 8509817 T<br>US 5801881 A | 28-02-1995<br>14-05-1998<br>29-08-1994<br>18-08-1994<br>22-11-1995<br>15-10-1996<br>01-09-1998 |
| EP 0732584 A | 18-09-1996 | US 5624845 A<br>US 5609744 A<br>US 5607568 A<br>US 5538898 A<br>JP 8256755 A | 29-04-1997<br>11-03-1997<br>04-03-1997<br>23-07-1996<br>08-10-1996 |
| WO 9631522 A | 10-10-1996 | US 5720928 A<br>AU 5532196 A<br>EP 0871640 A<br>JP 11503022 T | 24-02-1998<br>23-10-1996<br>21-10-1998<br>23-03-1999 |
| WO 9624082 A | 08-08-1996 | US 5671085 A<br>AU 4773896 A<br>CA 2210801 A<br>EP 0807273 A<br>JP 11501404 T | 23-09-1997<br>21-08-1996<br>08-08-1996<br>19-11-1997<br>02-02-1999 |
| US 4621911 A | 11-11-1986 | KEINE | |

EPO FORM P0461

Für nähere Einzelheiten zu diesem Anhang : siehe Amtsblatt des Europäischen Patentamts, Nr.12/82